(19) Europäisches Patentamt
European Patent Office
Office européen des brevets

(11) EP 2 644 617 A1

(12) EUROPEAN PATENT APPLICATION
published in accordance with Art. 153(4) EPC

(43) Date of publication:
**02.10.2013 Bulletin 2013/40**

(21) Application number: **10859902.8**

(22) Date of filing: **22.11.2010**

(51) Int Cl.:
***C07K 1/06*** (2006.01) ***C07H 21/00*** (2006.01)
***G06F 19/18*** (2011.01) ***C07B 47/00*** (2006.01)

(86) International application number:
**PCT/RU2010/000694**

(87) International publication number:
**WO 2012/070968 (31.05.2012 Gazette 2012/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(71) Applicants:
- **Farber, Boris Slavinovich**
  **Moscow 121151 (RU)**
- **Farber, Sof'ya Borisovna**
  **Moscow 121151 (RU)**

(72) Inventor: **MARTYNOV, Artur Viktorovich**
**Kharkov 61171 (UA)**

(74) Representative: **Modiano, Micaela Nadia**
**Modiano & Partners**
**Thierschstrasse 11**
**80538 München (DE)**

(54) **METHOD FOR THE MOLECULAR DESIGN AND SYNTHESIS OF THERAPEUTIC AND PREVENTIVE DRUGS**

(57) **Industrial Application:** This invention may be used in human and veterinary medicine for the design (creation and synthesis) of therapeutic and preventive drugs that are effective for the treatment of oncological and viral human and animal illnesses and for the design of new medicines.

**Summary of the Invention:** A new method of design and synthesis of therapeutic and preventive drugs in which a biopolymer target (protein, DNA, RNA, or a mixture of these) is used, and in the capacity of a ligand, the same biopolymer target is used, which is cut into oligomer fragments (nucleases, synthetic nucleases, and proteases); the fragments are modified through changing their charges to the opposite charge (acylation of anhydrides of dicarbonate acids or alkylation with halogen-carbonic acids). Also in the capacity of a ligand, the same biopolymer target is used, which is modified by partially changing the molecules' charges to the opposite with the creation of supramolecular biopolymer assemblies. We used supramolecule assemblies made from oligomers that were products of the hydrolysis of biopolymers, but with a change of the change of the molecules to the opposite charge, as well as the partial change of the charges of the target biopolymers.

**Technical Result:** A method of molecular design and synthesis of new, unique therapeutic and preventive drugs based on self-organizing systems. The application of the method will allow a significant cut to expenditures on the design and synthesis of new drugs, expand the activity spectra of existing protein gene-engineered drugs, and create new classes of dynamic therapeutic and preventive drugs that self-adapt to the organism and target.

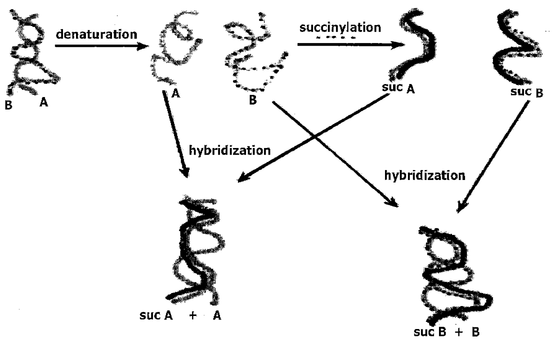


Fig. 4 (two sheets)



Printed by Jouve, 75001 PARIS (FR)

(Cont. next page)

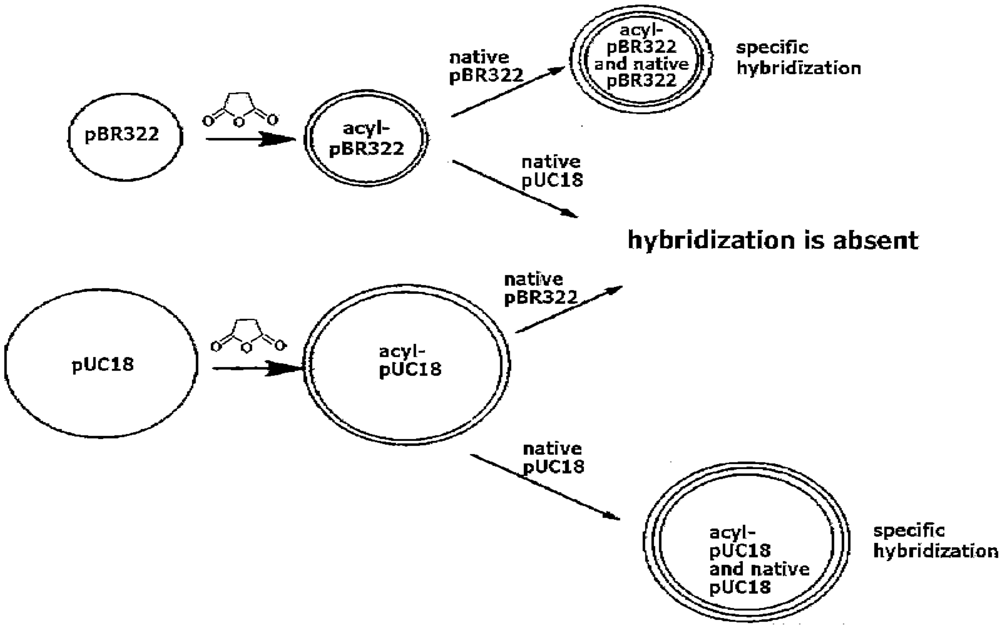


Fig. 4: Specific Hybridization of Acylated RNA Only with their Precursors (below: the same reaction with DNA plasmids

## Description

### Technical Field

This invention is related to medicine and pharmaceuticals, specifically to methods for designing and synthesizing new medicines.

### Previous Level of Technology

An important place in the modern structure of the pharmaceutical market belongs to drugs: biotechnological products of various origins. These are drugs that are essential to patients' lives, such as recombinant insulins, interferons, interleukines, erythropoietins, and so on. At the same time, statistical data on introducing these drugs on the market, as well as a number of low-molecular drugs, demonstrate that they are insignificantly more effective than a placebo. For example, the use of beta-interferon (in the treatment of disseminated sclerosis) exceeds the placebo in effectiveness by only 8 % (placebo: 30%; beta-interferon: 38%). The situation is almost the same for low-molecular drugs such as antihypertensives. The effectiveness of amlodipin at the third stage of clinical tests was only 22% higher than that of the placebo (amlodipin: 52%; placebo: 30%). The reasons that a drug might turn out to be ineffective or of little effect for 48% of patients have not yet been determined. The most difficult to explain is the ineffectiveness of drugs whose acceptors are cell receptors that have long been the subjects of study: namely, adreno-, cholino-, and histamine receptors. It remains a mystery why one and the same drug can be completely ineffective for one group of patience while remaining effective for another group. Due to this little-studied peculiarity of the human organism, the majority of antihypertensive drugs are taken in combination. It is especially necessary to combine at least three drugs with different mechanisms of action but the same final result: for example, antihypertensive or cytostatic. In the latter case, the differences between various kinds of tumors are especially marked, not only in sensitivity, but also in the individual peculiarities of a specific tumor and host organism. Even polychemotherapy often turns out to be ineffective in the treatment of patients with cancer. The FDA's depressing statistics on the third stage of clinical tests of drugs demonstrate the low effectiveness of practically all medicinal drugs available on the pharmaceutical market. The average effectiveness of the strongest drug (morphine) is 75%. In other cases, we observe either intolerance and toxic effects or an opposite reaction. Even in narcotic drug applications, only 60% of the people who took them are observed to experience the classical effects. The other group of people who took, for example, cocaine, suffered a severe headache and dizziness without any signs of anesthesia. This kind of divergence of effects may be caused by polymorphism of the receptor system within human population. Earlier, the structure of receptors was considered to be absolutely conservative and invariable for a single, or sometimes several, animal species. At present, more and more scientists are tending to believe that receptors differ in much the same way as do human faces, even within a single species. These differences are caused not only and not so much by the change of the primary amino acid sequence of receptors' protein base as by conformation changes of secondary and tertiary structures. Although they are formally similar in primary structure and molecular weight, different people's receptors are actually different combinations of protein isoforms. This is especially apparent in the example of major histocompatibility complex (MHC) antigen isoform combination. The selection of this complex is vitally important to organ transplantation processes. If thousands of variants and combinations exist for the MHC system, why should the structures of an organism's other receptors be conservative within a species? Most likely, similarly to MHC antigens, the tertiary structures of the majority of cell receptors differ significantly by isoform profile within a population. This hypothesis provides a good explanation for drugs' low levels of effectiveness. A conservative structure of a classical drug (like one "key") cannot match a specific receptor ("many different, though similar, locks") in all individuals of one species equally and with equal affinity.

In order to increase drug effectiveness, it is necessary to change the concept of drug development and approaches to this process. For example, docking[i], one of the most effective methods of modern drug design,[ii],[iii]uses a conservative sequence of one receptor. Sometimes target conformations are used in different solvents with further overlay of conformations. It will never be possible to obtain infinitely many receptors and find ONE inhibitor substance for all conformations. One of nature's successful solutions is immunoglobulin system evolution, which protects higher organisms from external aggressive factors, such as viruses, microorganisms, and fungi. Practically the same immunoglobulin base (Fc areas and heavy chains) with a large number of different variants on FAB- fragments being specific to their targets- is quite a successful solution.

In this situation, affinity to targets may vary from 5% of IgM to 95% of IgG. Sometimes one target antigen may cause the generation of several hundred thousand variants of immunoglobulins with different monoclonal specificity for different epitopes. This kind of polymorphism justifies itself: the majority of the population survives infectious diseases. In many cases, reconvalescent donor immunoglobulins are still the only effective means of treating many diseases, such as SARS and Marburg fever.

Following the logic of nature, we realize that choosing the classical method - synthesizing ONE compound to

treat one disease - is irrational and ineffective. This is proven by the decreasing amount of drugs with original structures introduced in the world every year.

To provide the maximum affinity for the maximum number of people, it is necessary to have a mixture of millions of molecules that are very similar but still different from each other in one vial. In this case we obtain not one "key," but a whole bunch of keys.

At least one "key" from this "bunch" will match a specific patient and his/her original receptor. If it is impracticable under modern conditions to synthesize a specific inhibitor for a specific patient, then the only option is to produce millions of inhibitor isoforms in one mole of substance.

In our opinion, one of the most reasonable methods of solving the problem of low drug effectiveness is to obtain precision partially chemically modified recombinant biotechnological preparations: biopolymers (proteins, polysaccharides, polynucleotides, tannins, self-organized structures/phospholipids, etc.). The usage of this technology will bring pharmaceutical science to a level of intensive development, make molecular modeling methods much simpler and increase the probability of a practical way out for the introduction of drugs.

Initial studies with a partial modification of the structure of the protein were performed with immunoglobulin; the results are presented below. As a rule, except for the primary structure of the compounds, the most important role is played by stereochemical properties. Here, the laws of randomness come into play. For example, chloromycetin has two stereoisomeres. Only one of these, the sinistrorotatory, is active.

The picture looks similar with the compounds newly corrected with the help of docking: a great affinity to the protein of the target in the model and the complete lack of activity in the synthesized connections. This is caused by the fact that ideal modeling conditions do not take into account the full variety of influences on the drug-target interaction process. These include parameters such as the temperature, nature, and properties of the solvent, the presence of assistive substances: the agents, gravitation, pressure, and, of course, the conformation of the substances. In the majority of cases, it is the stereochemical structure of the designed substance that does not coincide with the structure of the synthesized compound. Often, it is impossible to synthesize the necessary substance in general. In order to exclude incorrect conformations during modeling, compounds with a structure that has long been confirmed by physicochemical methods must be used in the capacity of reactants. A contradiction arises again: can this kind of longknown substance inhibit a new target? For these goals, "constructors" can be used: proteins and polynucleotides.

The former consist of long-studied L-amino acids, while the later consist of mononucleotides. Monomer structures are well-studied and -confirmed, as are their structures within a polymer and the influence of neighboring monomers on each other (this fact is taken into account and used in the process of design and synthesis of primers for a polymerase chain reaction).

Correspondingly, if we look for a drug among peptides and polynucleotides in advance and simply sort out a monomer sequence, we can find the necessary inhibitor or activator with a high degree of probability and also obtain its synthetic derivative.

In this situation, a number of problems arise: how to vary the hydrophobicity and charges of the oligomers obtained and how to protect them from the action of lytic factors in the organism: peptidases and nucleases. Additionally, these compounds must not be large in size; otherwise, they will not be able to get into cells and tissues. One of the methods for modifying protein structure in order to crystallize them better and study the X-ray structures is the acylation of terminal amino groups, lysines, and histidines, by means of various agents, among which the simplest are anhydrides of carboxylic (and polycarboxylic) acids: acetic, succinic, and maleic acids.

The majority of the protein crystals studied using the X-ray structural analysis method are completely succinylated derivatives.

Succinylation leads to a change in molecular charge through the substitution of negatively charged carboxylic groups for positively charged amino groups. If acylation is applied instead of succinylation, not only the molecular charge but also the molecular hydrophobicity level will change.

**Table 1: Structures of Modified Monomer Amino Acids Formed during Protein Acylation with Succinic Anhydride**

| No. | Monomer Structure | Monomer Name |
|---|---|---|
| | | |

(continued)

| 1 | 2 | 3 |
|---|---|---|
| 1 | 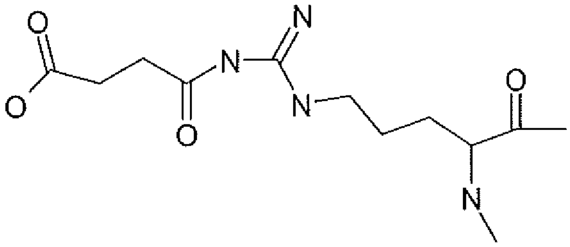 | |
| Agrin in succinamide 2 | 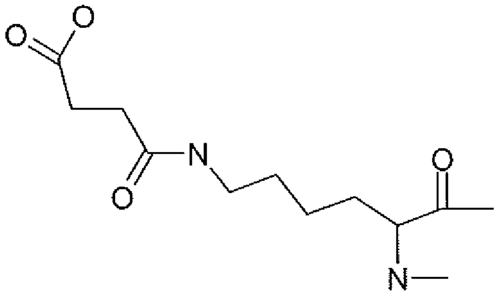 | |
| Lysine succinamide3 | 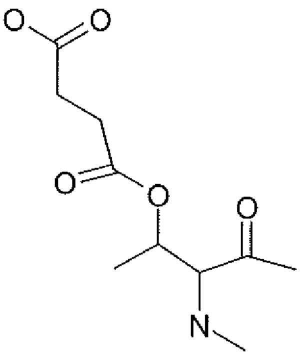 | |
| Ether of threonine and succinic acid4 | 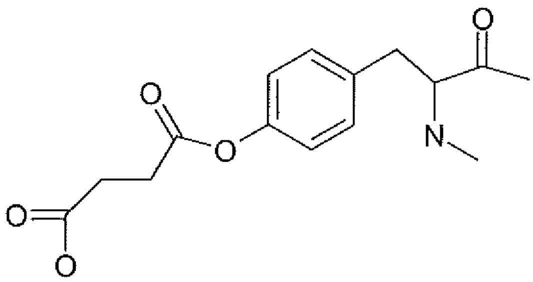 | |

Ether of tyrosine and succinic acidConformation changes of all amino acids in the acylation process by means of succinic and acetic anhydrides are quite well-studied; this eliminates a large portion of the random accidents that may occur during drug design. In this case, the approaches to modeling agonists (activators) and antagonists are completely different from the point of view of receptor polymorphism; this also concerns vaccine design.

The classic method for the search for and obtainment of drugs, which has been given the name of classic empirical screening, is known. This method is characterized by a certain succession of procedures: first, a few thousand chemical compounds are synthesized; the structure of the synthesized compounds is confirmed; a preliminary elimination of inactive compounds is conducted on in vitro screening models. The most active compounds are then studied in animal models. The deficiency of this method is the low percentage of production of active compounds: out of several tens of thousands of synthesized substances, one to two make it to the clinical research stage. It is possible that not one of the substances will make it to the production stage. In addition to the high volume of resources required for this approach, it is impossible to predict the side effects of synthesized compounds; substances that are highly active at first glance often turn out to be highly toxic. Dozens of years can be spent searching for a single active compound. All compounds synthesized according to this principle are xenobiotics; that is, they have never been encountered in nature before.

Accordingly, these substances will cause inestimable damage to the environment when they are excreted from the human body after treatment, since nature does not contain mechanisms to render these substances harmless. In addition, these substances are part of a group of static solid structures, which causes organisms to quickly adapt to the drug: adaptation of microorganisms to antibiotics, of viruses to anti-viral drugs and vaccinations, of tumors to anti-tumor drugs, and so on.[iv] For this same reason, the average effectiveness of xenobiotics is slightly higher than that of the placebo and rarely exceeds 50%.

Another approach to the design and synthesis of drugs is known; it has been conditionally named drag design. The method in [**Errore**. **Il segnalibro non è definito**.] is the prototype for the invention. It includes a series of procedures: first, the target for drug action is determined; as a rule, this is either a protein or glycoprotein receptor. The given protein is separated out, and its structure is established through the use of X-ray structural analysis and NMR spectroscopy. The active center of the protein or the interaction point with potential drugs is determined [v]. With the assistance of molecular computer modeling, the interaction between the target and several drug models is studied [vi], drug models are chosen with the most correspondence (affinity) by molecule structure and charge with the active center of the protein (receptor) and the target [vii]. Similar procedures also lead to other modifications of the drag design method [viii, ix]. Since this mixture of oligomer biopolymers is capable of adaptation and self-organization but is not able to reproduce like a live organism, it has been named a quasi-life system.

Then synthesis and other procedures are conducted as they are in the classic screening method. The advantage in comparison to screening is a significantly less resource-intensive drag design process: just a few dozen substances are synthesized with a significantly higher likelihood of obtainment of a truly effective drug (sometimes, up to 20% of the designed and synthesized connections possess the activity claimed). Many of the limitations of classical screening are also applicable to drag design: the high toxicity of the majority of products and the danger of their contamination of the environment, the high adaptability of an organism to a drug, and the ineffectiveness of the drug for up to 50% of the population in connection with the conservative nature of the structure. However, a new problem that has arisen during the use of drag design methods is the non-correspondence of the properties of the modeled and synthesized substances: highly active substances with a maximum level of affinity end up being inactive and toxic in practice.

**Disclosure of the Invention**

This invention was based on the task of developing a new method for the design and synthesis of new, highly effective (at an effectiveness in the population of near 100%) drugs that are quickly metabolized in the body and decomposed in nature based on dynamic systems (self-organizing and self-adapting to targets) with minimum expenditures on development.

**The task at hand is addressed** through a method of design and synthesis of therapeutic and preventive drugs, including the determination of the drug's target and, using molecular computer modeling methods, the study of the interaction between the target and several models of drug-ligands and the synthesis of the most active drug possible, distinguished by the fact that in the capacity of a ligand, the same biopolymer target is used, but it is cut into oligomer fragments, or the same, unchanged biopolymer target is used, modified by changing its molecular charge to the opposite charge, with the creation of supramolecular assemblies that are used as an active drug. The following are things that can be used in the capacity of a biopolymer target: proteins or protein mixtures, milk, egg white, DNA, RNA, or a mixture of DNA, RNA, proteins, and combinations thereof. The biopolymer target is cut in fragments with the help of proteaseses, nucleases, or synthetic nucleases. The biopolymer target fragments obtained are modified through changing their charge to the opposite charge through acylation with anhydrides of dicarbonate acids or alkylation with halogen-carbon acids. Also, the same biopolymer target may be used as a ligand, but in its native, unchanged state, which is then modified through partially changing the charges of the molecules to the opposite sign with the creation of supramolecular biopolymer assemblies.

We used a supramolecular assembly made of oligomers that were the products of biopolymer hydrolysis, but with molecules changed to the opposite sign, as well as with whole biopolymers that were partially changed to the opposite charge. "Assembly" is a term from supramolecular chemistry. The objects of supramolecular chemistry are supramolecular assemblies that build themselves out of their components; i.e. the current geometrical and chemical correspondence of fragments similar to the self-activated collection of the most complex spatial structures in a live cell [x,xi].

**Short Description of Drawings**

Fig. 1: Structure of $\alpha$-2b-Interferon according to Data from the X-Ray-Structural Analysis (online public database of the US National Library of Medicine, www.ncbi.nlm.nih.gov/entrez/ query.fcgi?db=Structure? 1RH2). Lysine, histidine, and arginine residues are depicted.

Fig. 2: Index of Anti-Proliferative Activity of IFN Derivatives toward CaOv Cells

Fig. 3: Index of antiproliferative activity of IFN derivatives toward SW-480 Cells

Fig. 4: Specific Hybridization of Acylated RNA only with Their Precursors (below: the same reaction with DNA plasmids)

Fig. 5: Replacement of a Hydrogen Bond with an Ionic Bond in the Creation of Hybrids between Anticanum and the Target

Fig. 6: Sarcoma. Hematoxylin-Eosin Dye

Fig. 7: Electronic Microphotography of the Influenza Virus (from www.news.wisc.edu/ newsphotos/influenza.html). The virus's polymorphism can clearly be seen in the virions of various sizes and shapes.

Fig. 8: Dependence between a Titer of Induced Specific Antibodies and the Acylation Level of a Corpuscular *Pseudomonas* Antigen

Fig. 9: Dependence between a Titer of Induced Specific Antibodies and the Acylation Level of a Soluble High-Molecular-Weight *Pseudomonas* Antigen

**Case Studies**

**Example 1: Synthesis of a Quasi-Life Structure Based on Partially Modified Alpha-Interferon**

The average effectiveness of alpha-interferon in the treatment of, for example, cervical cancer, is fairly low (about 15%). The reason for this is the inability of the interferon to interact with the cell receptors. In certain cases, this receptor is blocked by external factors; in other cases, this receptor differs from the ideal in connection with the cancerous transformation of the cells and their mutational changes. To discover the effects of the interferon, we must exchange one individual compound - alpha interferon (the key) - with a mixture of very similar, but distinct molecules of modified interferons (a bunch of skeleton keys). A selection of "skeleton keys" from hundreds of molecules of interferon can be done through a partial combinatory modification of the structure of alpha interferon.

For example, native alpha interferon contains contains eight fragments of lysine and three fragments of histidine, which can be acylated using succinic anhydride (Fig. 1). In order to obtain a "bunch of skeleton keys" a mixture of the maximum number of different interferon derivatives must be obtained in one volume with an insignificant change to the active center (area of connection with the receptor): from fully acylated to monosubstituted, mixed with each other in various molecular combinations. This requires determination of the level of substitution that would facilitate the maximum drug activity in the presence of the maximum quantity of various molecules in one volume of substance.

For alpha-interferon, this is a three-substituted polymorphic derivative. We specifically used the term "polymorphic" in connection with the fact that the solution actually contains 3!*8! interferon derivatives; it contains fully substituted and monosubstituted interferon and a mixture of derivatives with a level of mixed substitutions greater and less than three. Therefore, partial precision modification allows us to obtain a "bunch of skeleton keys" to a single polymorphic receptor, which significantly increases the pharmacological activity of the drug in a population of humans.

At minimum, one of the 3!*8! derivatives will correspond with a given receptor and the drug will be biologically active in the given specific instance. In addition, the appearance of these drugs may keep microorganisms and viruses from adapting to the drug. For example, this may take place in the case of bacteriocin modification. This modification may significantly expand the activity spectrum of bacteriocins and increase their protein breakdown stability; this will allow new peptide antibiotics to be created on their basis on an industrial scale.

For the synthesis of quasi- life systems on the basis of $\alpha$- 2b- interferon, interferon (IFN) (Biolek, Kharkiv, Ukraine) and succinic and aconitic anhydrides (Aldrich- Sigma, USA) were used. The acylated IFN was made in a weak alkaline buffer solution through adding powdered anhydride in the amounts presented in Tables 1 and 2. The end of the reaction was controlled through changing the molecular weight of the IFN through pulse- gel- electrophoresis in polyacrylamide gel, using non- acylated IFN as a control [B]. The gels were revealed through silver staining[xii].The modified IFN were cleaned with chromatography on Sephadex G- 25 and then preserved at- 20°C. Their anti- proliferative activity was studied on cells of the CaOv line, which is highly sensitive to interferons *in vitro,* and on SW- 480, a cell line with a low sensitivity to the activity of IFN *in vitro.* The cells were cultivated in a DMEM medium (Aldrich- Sigma, USA) with the addition of 10% FCS and 50 mcg/ml gentamycin (Aldrich- Sigma, USA) with a density of $10^4$ cells/$cm^2$ in 96- well plates (Bayer) at a temperature of 37°C in an atmosphere of 5% $CO_2$. The cell cultures' sensitivity to IFN was evaluated by the level of slowing of cell growth after treatment with the quasi- life IFN derivatives.

The Index of Anti-Proliferative Activity (IAA) of IFN (%) was calculated as a ratio between the number of cells in the experimental well and the number of cells in the control multiplied by 100. The non-specific cytotoxic effect was determined through dyeing the cells with trypan blue incubation after a 24-hour incubation of the cells with IFN derivatives. Not one of the interferon derivatives demonstrated non-specific cytotoxicity. The original titer of non-modified IFN was 2.5 x $10^3$ ME/ml; IFN and its quasi-life derivatives were added to the medium at a dose of 1000 U/ml (0.05 mcl/well); the acylated interferons were added in accordance with the calculation of the concentration of the protein. The cells were incubated for 72 hours with interferon. The number of cells was determined using an inverted microscope (Leica, Germany) every twelve hours.

Interferon is a low- molecular protein with a molecular weight of 8 kDa. It contains eight fragments of lysine and three fragments of histidine [xiii, xiv], which are capable of being acylated by succinic (SA) and aconitic (AA) anhydrides. In Fig. 1, the structure of α- 2b- interferon is shown.

As may be seen in Fig. 1, free lysines are found on the surface of the molecule and fulfill a buffering function. The receptor zone (the lower part of the IFN molecule in Fig. 1) contains only one lysine and histidine. The amino groups of these amino acids were inaccessible for acylation by anhydrides. The remainders of histidine from the regulatory area of the IFN molecule (the upper part of the molecule in Fig. 1), conversely, take part in the creation of interferon's internal structure. Thus, the histidine amino groups will not take part in the acylation reaction in connection with the steric hindrances. However, the hydroxyl groups of amino acids like threonine and serine can be acylated, as can sulfhydryl amino acids. To prevent the passing of the reaction to the hydroxylic and sulfhydrylic groups, the acylation reaction was conducted in a weakly alkaline medium. At a pH of more than 7.5, deprotonation of free lysine amino groups is observed, and the lysines are significantly stronger nucleophiles than are the hydroxyls and the sulfo groups. Accordingly, when an exact calculation is made of the relationship between IFN and the anhydrides, seven derivatives each may be obtained in whose molecules from one to seven lysine amino groups will be acylated. Tables 1 and 2 present the relationship between the reagents and the results of the analysis of synthesized succinimide and aconitine derivatives of α-2b-interferon.

Table 1: Results of the Analysis of Succinimide Derivatives of α-2b-Interferon (SIF)

| **Mole Ratio IFN to Anhydride** | **Rf** | **Mr, Da** | |
|---|---|---|---|
| | | **Calculated** | **Determined** |
| 1:1 | 0.490±0.005 | 18359±10 | 18360±50 |
| 1:2 | 0.462±0.005 | 18458±10 | 18460±50 |
| 1:3 | 0.460±0.005 | 18557±10 | 18560+50 |
| 1:4 | 0.456±0.005 | 18656±10 | 18655±50 |
| 1:5 | 0.442±0.005 | 18755±10 | 18750±50 |
| 1:6 | 0.440±0.005 | 18854±10 | 18855±50 |
| 1:7 | 0.436±0.005 | 18953±10 | 18920±50 |
| 0 (Control interferon) | 0.492±0.005 | 18260±10 | 18250±50 |

Table 2: Results of the Analysis of Aconitine Derivatives of α-2b-Interferon (AIF)

| **Mole Ratio IFN to Anhydride** | **Rf** | **Mr, Da** | |
|---|---|---|---|
| | | **Calculated** | **Determined** |
| 1:1 | 0.489±0.005 | 18280 ±10 | 18300 ±50 |
| 1:2 | 0.463±0.005 | 18457 ±10 | 18470 ±50 |
| 1:3 | 0.461±0.005 | 18555 ±10 | 18580 ±50 |
| 1:4 | 0.455±0.005 | 18634 ±10 | 18640 ±50 |
| 1:5 | 0.443±0.005 | 18754 ±10 | 18760 ±50 |
| 1:6 | 0.441±0.005 | 18857 ±10 | 18860 ±50 |
| 1:7 | 0.438±0.005 | 18956 ±10 | 18980 ±50 |

(continued)

| Mole Ratio IFN to Anhydride | Rf | Mr, Da | |
|---|---|---|---|
| | | Calculated | Determined |
| 0 (Control interferon) | 0.492±0.005 | 18260 ±10 | 18250 ±50 |

As may be seen in the table, the calculated and established masses do not differ from one another, which bears witness to the conclusion of the acylation reaction both in the synthesis of succinic and in the group of aconitic IFN derivatives.

The change in the anti-proliferative effect of the IFN may occur dependent either on its level of affinity with the cell membrane or on the change of the structure of the molecule itself and the corresponding arising of new, previously unknown effects (as phospholipidation of proteins sometimes increases their biological activity by many values of ten). Two cultures of human tumor cells were taken for study: one with a high level of sensitivity to interferon and one with a low level of interferon sensitivity. In Figs. 2 and 3, the results of the study of the anti-proliferative activity of the aconitic and succinic IFN derivatives may be seen.

As may be seen in Fig. 2, the acylation of one lysine in the IFN structure by succinic anhydride leads to an increase in the index of anti-proliferation activity (IAA) by 3.2 times. In the derivatives with 2-5 substituted lysines, IAA continues to decrease, and the anti-proliferative activity does not appear in 6-substituted SIFs at all. A slightly different picture is seen with the aconitine IFN derivatives: the acylation of one lysine in the IFN by aconitine anhydride leads to an increase in anti-proliferation effect of 3.6 times, while similar activity is not seen in the 4-substituted derivatives.

For the SW-480 cultures with low sensitivity to interferon, a different picture can be seen (Fig. 3): the unmodified interferon does not have anti-proliferative activity, while the maximum activity is seen in the derivatives with 3 substituted lysines. The 3-substituted AIF had an activity level 16% higher than that of the SIF. A plateau of stability from three to five substituted lysines was seen in the SIF, while that of the AIF titer began to fall with the substitution of the next lysine.

Thus a certain pattern is observed that is less likely characteristic of cultures of inhibited cells than of groups of derivative IFN: the largest amount of anti-proliferative activity was shown by aconitine IFN derivative with 1 substituted lysine and three for SW-480 cultures. Although the succinic derivatives were less active, many of the derivatives maintained a high level of activity up through 6-7 substitutions. When a switch was made to a culture that was not sensitive to interferon, a leap was observed in maximum activity in the less acidic derivatives. It is interesting that cell division was fully inhibited just twelve hours after the introduction of acylated interferons to the culture (within the next 60 hours, the number of cells did not increase). In certain live cells, cytological changes were observed that are characteristic of apoptosis: fragmentation of the cell nucleus and karyolysis. A slow in growth in more than 95% of the cells was observed after just twelve hours of incubation, whereas the control IFN in the same concentration was literally ineffective.

Some conclusions may therefore be drawn:

1 The acylation of one lysine in $\alpha$-2b-interferon in cis-aconitic and succinic anhydrides led to a 3-3.5-fold increase in the anti-proliferative activity of interferon in a sensitive culture of a CaOV ovarian cystadenocarcinoma.

2 The largest anti-proliferative activity was demonstrated against cultures of large intestinal SW-480 cancer that have a low sensitivity to native interferon by three-substituted lysine derivatives, both among the aconitic and the succinic derivatives.

3 Acylated interferon leads to a broadening of the spectrum of its anti-proliferative activity on non-sensitive cultures of cancer cells.

4 The maximum anti-proliferative effect by the acylated interferons on both sensitive and insensitive cancer cell cultures arose after just twelve hours at a 95% index of anti-proliferative activity (for 1- and 3-substituted derivatives accordingly).

5 Interferons that are acylated using aconitic and succinic anhydride are a new class of promising anti-proliferative substances, and they require more intense and broader study on *in vivo* models.

Table 1: Results of the Analysis of Succinimide Derivatives of $\alpha$-2b-Interferon (SIF)

| Mole Ratio IFN to Anhydride | Rf | Mr, Da | |
|---|---|---|---|
| | | Calculated | Determined |
| 1:1 | 0.490±0.005 | 18359±10 | 18360±50 |
| 1:2 | 0.462±0.005 | 18458±10 | 18460±50 |

(continued)

| Mole Ratio IFN to Anhydride | Rf | Mr, Da | |
|---|---|---|---|
| | | Calculated | Determined |
| 1:3 | 0.460±0.005 | 18557±10 | 18560±50 |
| 1:4 | 0.456±0.005 | 18656±10 | 18655±50 |
| 1:5 | 0.442±0.005 | 18755±10 | 18750±50 |
| 1:6 | 0.440±0.005 | 18854±10 | 18855±50 |
| 1:7 | 0.436±0.005 | 18953±10 | 18920±50 |
| 0 (Control interferon) | 0.492±0.005 | 18260±10 | 18250±50 |

Table 2: Results of the Analysis of Aconitine Derivatives of α-2b-Interferon (AIF)

| Mole Ratio IFN to Anhydride | Rf | Mr, Da | |
|---|---|---|---|
| | | Calculated | Determined |
| 1:1 | 0.489±0.005 | 18280 ±10 | 18300 ±50 |
| 1:2 | 0.463±0.005 | 18457 ±10 | 18470 ±50 |
| 1:3 | 0.461±0.005 | 18555 ±10 | 19580 ±50 |
| 1:4 | 0.455±0.005 | 18634 ±10 | 18640 ±50 |
| 1:5 | 0.443±0.005 | 18754 ±10 | 18760 ±50 |
| 1:6 | 0.441±0.005 | 18857 ±10 | 18860 ±50 |
| 1:7 | 0.438±0.005 | 18956 ±10 | 18980 ±50 |
| 0 (Control interferon) | 0.492±0.005 | 18260 ±10 | 18250 ±50 |

**Example 2: The Design of the Anticanum Self-Organizing Quasi-Life System with AntiCancer Properties Based on Modified Oligonucleotides**

Among the existing new fields in the treatment of oncological diseases, there are several quite promising approaches. One of these approaches may be considered the development of drugs for cancer gene therapy [xv] . In this approach, the main active principle is polynucleotides. Gene therapy can be divided into two groups: means to inactivate genes [xvi] and means for introducing genetic material into a cell [xvii] . Gene inactivators are also called antisense polynucleotides [xviii] . Many research projects conducted in this direction have not come up with truly effective in vivo drugs. This is connected with a whole host of problems: the synthesized DNA (RNA) was quickly destroyed by blood nucleases [xix], did not penetrate the cells, and the genome repair systems were disrupted [xx] . Sometimes, a short- term block of the expression of protein targets and buildup in the hepatocytes has been observed [xxi] . There are various approaches to the design of oligonucleotide antisense, but the principle of their interaction with the target remains the same: the creation of hydrogen bonds between complementary nucleotides upon increasing the level of resistance to nuclease [xxii] . In certain cases, developers have protected the 5' end of an oligonucleotide from nuclease action; in other cases, they have modified the 3' end l[xxiii,xxiv] . A VI- biopharma's developers have exchanged deoxyribosil remnants for fragments of morpholine with the goal of creating fragments of DNA (RNA) that can withstand nuclease action [xxv, xxvi] . The principle of gene inactivation through their complementary interaction with antisense nucleotides has remained the same: the creation of hydrogen bonds. It is this hydrogen bond that is the main reason for the ineffectiveness of existing drugs based on antisense DNA (RNA) . Ferments of a type of helicase [xxvii] very quickly and easily unwind antisense DNA that is hybridized with the gene target, and the gene's activity begins again. In our earlier studies [**Errore. Il segnalibro non è definito.**], when researching acylated polynucleotides, we discovered a new phenomenon: polynucleotides that had been acylated along exocyclic amino groups hybridize selectively only with their own non- acylated precursors. The pUC18 plasmid hybridized only with its acylated derivatives, while the pBR322 plasmid hybridized, accordingly, only with acylated pBR322 (Fig. 4) . Insoluble, non- melting conjugates were created. It was the non-melting property that distinguished the classic, double- helix DNA from the hybrids that had been obtained. They would not melt at any temperature and would not dissolve in anything except in concentrated alkalis. We explain this property

of the synthesized acyl- DNA by a change to the very principle of bonding between DNA chains: from hydrogen to ionic and mixed.

This bond (Fig. 5) was not stipulated at all by the natural repair mechanisms. Thus the cell helicases and nucleases will be ineffective if hybrids are created between this type of acyl-DNA (RNA) and its non-acylated predecessors. In addition to the polynucleotide phenomenon, we also saw a dependence between charge and activity in a series of other acylated biopolymers: proteins, polysaccharides, polynucleotides, tannins, bacteriophages, and immunoglobulins. On the basis of this research, a new veterinary antiviral drug was developed and implemented [xxviii]. We established that a certain precision modification to the structure of a biopolymer is capable of increasing its biological activity, fully changing its properties, or leading to the creation of self-organizing structures. The behaviors we discovered were taken as the basis for a principle of obtaining microRNA from acylated exocyclic amino groups. This drug was named Anticanum.

This microRNA is a new class of uncoded RNA with a length of 18-25 nucleotides, which negatively regulate post-translation gene expression. The mechanism of the activity of these small fragments of RNA is based on the interaction (hybridization) of microRNA with matrix RNA directly in the polyribosome complex. This hybridization will lead to the cessation of the synthesis of a specific protein. The role of microRNA in carcinogenesis and perspectives on using microRNA in cancer therapy is presented in a review [xxix]. However, microRNA is not capable of penetrating a cell membrane on its own. Currently, research is being done on the creation of various transportation systems for the delivery of microRNA to cell targets. The most promising carriers are liposomes, polymer nanoparticles, and self-organizing polymers [**Errore**. **Il segnalibro non è definito.**].

The ability of many adenocarcinomas to pick up oligonucleotides and nanoparticles by pinocytosis from the intercellular matrix is known[xxx,xxxi] . Healthy cells are not capable of picking up small oligonucleotides and liposomes [xxxii] . This facilitates Anticanum aggregation selectivity in cancerous cells and the drug’s lack of toxicity. In order to obtain Anticanum, we used accumulated RNA from yeast, fragmented pancreatic nuclease to fragments from 2- 15 n in size. Then the exocyclic amino groups of these oligonucleotides were modified by changing their charges. For purposes of improving Anticanum’s aggregation in tumors, it was included in monolamellar phosphatidylcholine liposomes. The selective aggregation of Anticanum in cancer cells leads to its hybridization with complementary targets in the matrix RNA of the cancer cell and to the gradual cessation of protein synthesis. Anticanum blocks not only the intronic, but also the exonic areas of matrix RNA, which leads to the practically instantaneous stoppage of protein synthesis. Anticanum’s activity is based on the inducement of apoptosis through the stoppage of protein synthesis. Anticanum does not affect healthy cells, blocks the synthesis of all cell proteins, and excludes the possibility of cancerous tumors’ adaptation to therapy and the selection of stable cells.

The molecular mechanism of the drug’s activity has not been studied.

In addition to showing anti-cancerous activities *in vitro,* Anticanum also showed a high activity *in vivo* on models of benzidine sarcomas in rats and Ehrlich’s ascites adenocarcinoma in mice (presented later in the report).

**Example 3: Obtaining the Substance of Anticanum.**

The microbial biomass contains up to 11% nucleic acids and may serve as a raw material for the obtainment of microbial RNA, on the basis of which it is possible to obtain derinates that are a mixture of oligonucleotides. The object of study for this stage of the work was RNA taken from *Saccharomyces cerevisiae* yeast biomass.

**Separation of Summary RNA from Baker’s Yeast.** Four kg of compressed yeast was thawed at room temperature. It was ground and suspended in 81 of boiling water containing 300 g of so-dium dodecal sulfate. The suspension was boiled for 40 minutes while being stirred constantly. It was then poured into steel centrifuge cups, which were quickly placed in ice and cooled to 5 °C (~15 min.), after which they were centrifuge in a 6K15 German-made centrifuge (17000 g, 10 min, 4°C). The sediment and a part of the gel-like interphase were removed, and the RNA that had migrated to the supernatant was separated out. This was accomplished by adding NaCl to the supernatant obtained in the previous stage, until a final concentration of 3 M was reached. After dissolving the salt, the suspension was left for 1 hour to allow the formation of sediment, which was then separated by a centrifuge at 17000 g over 10 min. The sediment was rinsed with two portions of 813 M NaCl each, suspended in 2 1 of ethanol, and left overnight. The next day, the suspension was centrifuge at 17000 g over the course of 10 minutes. The sediment was dissolved in distilled water to an RNA concentration of 450 D260 U/ml (~1.6 1). The solution was clarified by centrifuging under the same conditions, after which the RNA was precipitated from the supernatant through adding NaCl to a final concentration of 0.15 M and an equal volume of ethanol (~2 1). The sediment formed was separated from the supernatant through centrifuging (17000 g, 10 min), cleaned in 1 I of ethanol, and dehydrated in a CaCl vacuum desiccator. All operations were conducted at a temperature of 0-4 °C.

**Separation of High-Polymer RNA from Baker’s Yeast**

**Day 1: RNA Extraction.** 7.5 g of sodium dodecal sulfate were dissolved in 300 ml of water in a heat-resistant

one-liter glass beaker. The solution was brought to a boil, and over a period of 5 minutes, 30 g of dry yeast were added in such a manner that the temperature of the suspension did not fall below 98° C. The suspension obtained was boiled for 40 minutes while being stirred constantly, and boiling water was added to keep the mixture at 300 ml as the original water boiled off. At the end of the extraction, the suspension was cooled to approximately 60° C. Freshly boiled water was added to a volume of 450 ml. This was mixed, poured into a 500-ml. measuring cylinder, and left to settle at a temperature of 20° C for 22 hours.

**Day 2: RNA Desalting and Rinsing the Desalted RNA with 3 M NaCl.** The cooled supernatant (280 ml) was transferred to a measured glass beaker with a volume of 500 ml. 81 g of NaCl was added to it, as was water, to make a total of 450 ml. This was mixed and left to settle at a temperature of 19° C for 6 hours. After 6 hours, the interphase formed (250 ml) was bled off, and to the desalted RNA that was divided into two fractions (one part deposit, one part supernatant) was added 3 M NaCl up to 450 ml. This was mixed and left to settle overnight at a temperature of 19° C.

**Day 3: Rinsing the Desalted RNA with 3 M NaCl.**

The interphase (300 ml) was bled off, and 3 M NaCl was added to the desalted RNA up to 450 ml. This was mixed and left to settle at a temperature of 19° C for 5 hours. After 5 hours, the in-terphase (280 ml) was bled off, and 3 M NaCl was added to the desalted RNA up to 450 ml. This was mixed and left to settle at a temperature of 19° C for 3 hours. After 3 hours, the interphase (250 ml) was bled off, and 3 M NaCl was added to the desalted RNA up to 450 ml; this was mixed and left to settle overnight at a temperature of 19° C.

**Day 4: Rinsing the Desalted RNA with Alcohol.** There is almost no top layer. Sediment occupies a volume of ~100 ml. The supernatant was removed, and ethanol was added to the sediment up to a volume of 300 ml. This was mixed and left to settle at a temperature of 18° C for five hours. After five hours, the supernatant was poured off, and a fresh portion of ethanol was added to the 140 ml of sediment up to a volume of 320 ml. This was mixed and left to settle at a temperature of 19° C for 40 minutes, after which the supernatant was poured off. 120 ml of fresh ethanol was added to the 120 ml of sediment. This was mixed, and the supernatant was poured off after the mixture had been left to stand for 30 min. To the sediment (110 ml) was added 110 ml of fresh ethanol. This was mixed, and after 30 minutes of settling, the supernatant was poured off, and the RNA suspension was poured in a thin layer into a flat pan and air-dried at room temperature until the odor of alcohol had disappeared. The pure high-polymer RNA from the intermediate product was removed through water extraction in a cellophane dialysis bag.

**Enzymatic Splitting of the Total RNA Obtained.** In the capacity of a nuclease, pancreatic ribonuclease (RNAase) with an activity of 14000 U/mg in a quantity of 0.4% of the mass of the RNA was used. The RNA splitting was conducted over a period of four hours. The hydrolyzate was dehydrated in a flash drier.

**Chemical Modification of the Hydrolyzate's Oligonucleotides.** A 3.5% water solution of the hydrolyzate was obtained; succinic anhydride was added in a quantity of 10-45% of the dry weight of the hydrolyzate; this was mixed in the cold until the anhydride was fully dissolved. The solution obtained was sterilized for 120 with flowing steam. The prepared solution was studied further for presence of anti-cancerous properties.

**Example 4: A Study of Anticanum's Toxicity Levels**

Thirty- six series of experiments were conducted on 220 animals. The average lethal intravenous dose of Anticanum was established for three types of animals: non- speciated white mice weighing 20- 22 g, white rats weighing 190- 200 g, and guinea pigs weighing 300- 400 g of both sexes. With the goal of comparing the spectrum of therapeutic activity of Anticanum and the comparison drug, Taxotere, we also established the average lethal intravenous dose of Taxotere.

The calculation of the average lethal dose was made according to B. M. Shtabskiy's methods with the use of the equation:

$$X = \frac{Y-a}{a} \qquad (1)$$

Where Y-% of effect

$$a = \frac{Y_1 - Y_2}{X_1 - X_2}$$

$$b = \frac{\sum Y - a \sum X}{n}$$

where:

X1 and X2 are the values of the two extreme of the three doses studied that have an effect on more or less than 50% of the animals; the third dose is the intermediate;

Y1 и Y2 are the lethality percentages corresponding to doses X1 and X2;
∑Y- is the sum of the three doses studied;
the number of doses used in the calculations is equal to 3.

If we add to formula (1) a value of Y that is 50, 84, and 16 percent mortality, $LD_{50}$, $LD_{84}$, and $LD_{16}$ are calculated. Then d, the average margin of error for the average lethal dose was found through use of the Miller-Tainter formula:

$$m = \frac{2\ \delta}{\sqrt{2N}} \quad 2\text{-}\ LD_{84}\text{-}\ LD_{16};$$

N is the total number of animals in the groups in which even one animal died or survived, and the confidence ranges were established.

The mice were dosed by a slow intravenous method with Anticanum (vector liposomes were prepared before use: a 0.9% solution of NaCl was added to the dry lyophilized Anticanum powder that was taken in corresponding dosages) in dosages of 1000, 1500, 2000, 2500, and 3500 mcg/kg animal body weight. An analysis of the results indicates that the average lethal dosage of the drug for this species of animal is 2780 mcg/kg.

A study of Anticanum in rats at dosages of 1000, 2000, 2500, 3000, 3500, and 4000 mcg/kg provided the opportunity to determine that the average lethal dosage for this species of animal is 2590 mcg/kg. The average lethal dosage of Anticanum for guinea pigs is 2420 mcg/kg weight.

Intravenous Taxotere in rats showed LD 50 = 120 mcg/kg body weight.

A comparison of the characteristics of Anticanum and Taxotere is presented in Table 4.

**Table 4: Comparative Characteristics of Anticanum and Taxotere**

| Drug | ED50, mcg/kg | LD 50, mcg/kg in Rats, Intravenous Introduction | Therapeutic Index | Relative Therapeutic Index |
|---|---|---|---|---|
| Anticanum | 5 | 2590 | 518 | 151.5 |
| Taxotere | 35 | 120 | 3.42 | 1 |

An analysis of the data obtained, which are presented in Table 4, leads to the conclusion that Anticanum in liposomes is less toxic than the drug Taxotere, and exceeds that drug in comparison of effective dosage. The therapeutic index that characterizes the breadth of therapeutic activity was 151.5 times more for Anticanum than in the comparison drug due to the liposomal form. In order to extrapolate Anticanum's toxicity in humans, we calculated a species sensitivity ratio (SSR) according to the formula:

$$SSR = \frac{LD50\ (\text{for rats})}{LD50\ (\text{for mice})} = \frac{2590}{2780} = 0.9$$

Thus Anticanum does not have a species-related sensitivity, or it appears slowly.

A determination of Anticanum's cytotoxicity was made in a cultlure of HeLa-2 cells. For this purpose, 2-12 mcg of Anticanum per ml of medium were added to the 199 medium. A culture without Anticanum in it was used as a control. Cultures were observed daily over the course of five days. The Minimum Active Dose (MAD) of Anticanum was also considered to be the minimum amount of the drug that caused a degeneration of 90-95% of the cells (Table 5)

**Table 5: Comparative Sensitivity Characteristics of Cultures of HeLa-2 Tumor Cells to Anticanum**

| ---------------- | | Anticanum Activity at Various Acidity Levels among 199[1] Cell Cultures. | |
|---|---|---|---|
| Drug | MAD in mcg/ml | Control | Experiment |
| Anticanum | 10 | 0 | ++++ |
| Taxotere | 10 | 0 | ++ |
| Bare Liposomes | ------- | 0 | 0 |
| [1] Cytopathic activity;<br>++++ degeneration of 100% of the cells<br>0 lack of degeneration. | | | |

In establishing the minimum concentration of Anticanum that will slow the growth of cells, a comparison was made between the number of surviving cells and the concentration of Anticanum in the solution.

**Table 6: The Effect of Anticanum on HeLa Cells**

| Dose, mcg/ml | Number of Cells before Incubation, Millions | Number of Live Cells after Incubation, Millions, ±1000 | | Number of Live Cells after Incubation, % | |
|---|---|---|---|---|---|
| | | Anticanum | Taxotere | Anticanum | Taxotere |
| 2 | 150000±1000 | 72000 | 150000 | 48 | 100 |
| 4 | 153000±1000 | 21400 | 150000 | 14 | 98 |
| 6 | 150000±1200 | 9800 | 145000 | 6.5 | 97 |
| 8 | 152000±1000 | 0 | 135000 | 0 | 89 |
| 10 | 158000±1000 | 0 | 130000 | 0 | 82 |
| 12 | 162000±1000 | 0 | 153000 | 0 | 94 |

As may be seen in Table 6, an effective dose of Anticanum is between 8-12 mcg/ml solution.

Anticanum led to a 95% degeneration of tumor cells. To confirm the in vivo anti-tumor activity, Anticanum was studied in benzidine skin sarcoma and reinjected ascites adenocarcinomas in Barbados mice. In five mice with adenocarcinomas, the distribution of the Anticanum liposome throughout the animals' bodies was also studied using a fluorescent probe dissolved in a phospholipid layer.

**Example 5: A Study of the Anti-Cancer Activity of Anticanum on Benzidine Sarcoma.**

Before applying it to the silica gel, 7 ml of a solution of 2% benzidine and 0.9% sodium chloride was added until an opalescent suspension was formed (1 g silica gel for 5 ml NaCl solution). Twenty-five Barbados mice of both sexes with a weight of 18-20 g that were kept on a vivarium diet were administered benzidine and phorbol acetate immobilized on silica gel subcutaneously near the neck. After two weeks, 18 animals had developed tumors of different sizes in the form of a small bump on the neck near the silica gel granulomas. Each group of animals was administered the corresponding compound parenterally at a dose of 100 mcg/kg weight twice a day for two weeks, beginning at 16 days after administration of the carcinogen.

**Table 7: A Comparison of the Anti-Tumor Activity of Anticanum in Comparison to the Combination of an Analog (Taxotere) and a Lipid**

| Drug Name | Weight of Animal (g) | |
|---|---|---|
| | Before Treatment | After Treatment |
| Taxotere | 28 ±1.2 | 23± 1.1 (2 mice died) |
| Anticanum | 25± 1.7 | 15± 1.5 |
| Bare Liposomes | 26± 2.1 | 34 ±1.3 (5 mice died) |
| Note: n=7, p>0.05 in comparison with the control and previous data. | | |

As may be seen in Table 7, Anticanum decreased the weight of the experimental animals by 10 g; the control animals' weight continued to increase, and some of them died. After the dissection of the silica gel granulomas, it was established that the animals treated with Anticanum did not show signs that the granulomas had turned into malignant sarcomas.

The animals' survival rates are presented in Table 8.

**Table 8: Survival Rates of Animals with Benzidine Skin Sarcoma**

| Drug Name | Animal Survival, Days |
|---|---|
| Taxotere | 28±1.1 |
| Anticanum | 49±1.2 |
| Bare Liposomes | 17±0.9 |
| Note: n=10, p>0.05 in comparison with the control and previous data. | |

Thus Anticanum prolongs the life of animals twice as long as does Taxotere.

**Example 6: A Study of the Anti-Tumor Activity of Anticanum on Ehrlich's Ascites Adenocarcinoma**

The anti-tumor activity of the compositions were studied in models of Ehrlich's ascites carcinoma in young Barbados mice of both sexes with weights between 15-17 g (68 individuals), which were kept on a vivarium diet.

45 mice were inoculated from a mouse with adenocarcinoma using an insulin syringe with 0.1 ml ascitic fluid in the region of the liver. Within seven days, 42 mice showed signs of tumors (the body weight and belly size increased); two mice died on the second day; one mouse did not show signs of a tumor.

Ten mice were administered Anticanum in liposome form (see Table 4). Ten more mice were administered the Anticanum substance, and ten more were administered a 0.9% solution of sodium chloride with lipids.

**Table 9: Qualitative Biological and Statistical Characteristics in the Study of Anticanum's Anti-Tumor Activity.**

| **Substance** | **Liposomes** | **Time of Death of Animals after the First Injection. Average Value Days** | |
|---|---|---|---|
| | | **Experimental Animals** | **Control Animals** |
| **Anticanum** | + | 37.4±0.88 | 3.2±0.44 |
| **-//-** | - | 18±3.2 | 3.1±0.48 |
| **Taxotere** | + | 15±0.5 | 3±0.5 |
| -//- | - | 14±0.12 | 3±0.6 |
| Note: n=10, p>0.05 in comparison with the control and previous data. | | | |

Anticanum was given to those mice from which blood was drawn. Mice with Ehrlich's adenocarcinoma, after being given the tumor and treated, lived for 18 days when administered the Anticanum substance, which is 6 times longer than the control, and 37 days when administered Anticanum in liposomes, which is 12 times longer than the control. At an accuracy level of more than 99.5%, we can confirm a significant increase in anticarcinogenic activity in

liposomal Anticanum over the control, Taxotere. After dissection of the animals, signs of tumors and metastasis were not found in their bodies.

**Example 7: Study of the Distribution of the Anticanum Liposome throughout the Bodies of Animals**

For the study of the distribution of the liposomes throughout animals' organs, biologically inert 5,6-benzocoumarin was used, which is lipophilic and has fluorescent properties. The benzocoumarin was diluted in alcohol (0.5 M solution) and added to a phospholipid solution upon creation of the liposome in the quantity of 0.01 ml coumarin solution in 10 ml of the phospholipid solution. The most fluorescence was observed in the liver and ascitic fluids, which confirms the liposome's affinity for the tumor. In the control, the liposomes were distributed in the spleen, liver, and lymph nodes.

The distribution of the liposomes was studied in the bodies of mice with tumors (five animals) and healthy rats (five animals) after a fluid administration of Anticanum. Anticanum was administered at a dosage of 100 mcg/kg. After five hours, the mice were pithed and the fluorescence intensity was studied using an HP-F-40M fluorometer.

**Table 10: Accumulation of Anticanum Dependent on Speed of Introduction.**

| Animals | Organ | Form of Anticanum Introduction | Fluorescence, % * |
|---|---|---|---|
| Healthy Rats | | Slow Infusion | |
| -\|\|- | Plasma | | 23.4±0.1 |
| -\|\|- | Liver | | 26.1±0.1 |
| -\|\|- | Lungs | | 0.5±0.1 |
| -\|\|- | Spleen | | 6.2±0.1 |
| -\|\|- | Kidneys | | 32.2±0.1 |
| -\|\|- | Blood | | 25.6±0.1 |
| | * intravenous injection | | |
| -\|\|- | Plasma | | 25.3±0.1 |
| -\|\|- | Liver | | 56.2±0.1 |
| -\|\|- | Lungs | | 0.5+0.1 |
| -\|\|- | Spleen | | 0.3±0.1 |
| -\|\|- | Kidneys | | 0.2±0.1 |
| -\|\|- | Blood | | 5.5±0.1 |
| Mice with Adenocarcinoma | Slow Infusion | | |
| -\|\|- | Plasma | | 15.2±0.1 |
| -\|\|- | Liver | | 12.2±0.1 |
| -\|\|- | Lungs | | 0.5±0.1 |
| -\|\|- | Spleen | | 8.2±0.1 |
| -\|\|- | Kidneys | | 52.2±0.1 |
| -\|\|- | Ascitic Fluid | | 45.6±0.1 |
| | Fast Intravenous Introduction | | |
| -\|\|- | Plasma | | 23.2±0.1 |
| -\|\|- | Liver | | 60.2±0.1 |
| -\|\|- | Lungs | | 0.2±0.1 |

(continued)

| | Fast Intravenous Introduction | | |
|---|---|---|---|
| -\|\|- | Spleen | | 0.5±0.1 |
| -\|\|- | Kidneys | | 0.3±0.1 |
| -\|\|- | Ascitic Fluid | | 5.2±0.1 |
| * relative fluorescence: the percentage of fluorescence relative to the intensity of the fluorescence of the administered Anticanum solution. | | | |

As can be seen in the table, when a tumor is present, the aggregation of Anticanum goes there, but in dependence on the time taken for the infusion. The faster Anticanum is introduced, the more of it is collected in the liver. It is therefore rational to introduce Anticanum via slow infusion.

**Example 8: A Method for Obtaining Self-Organizing Systems from a Base of a Group of Modified Peptides with Anti-Viral Properties (the drug Albuvir)**

500 mg of dry, nonfat milk is sprinkled into 50 ml of distilled water and the pH is brought to 8.0 using a sodium hydroxide solution. To the protein solution obtained, trypsin immobilized on caproic granules is added at a ferment: substrate ratio of 1:100 in a 0.1 M phosphate buffer solution. This is constantly stirred for four hours. Then the immobilized ferment is removed through filtration. To the supernatant fluid peptide mixture obtained, 750 mg of solid succinic anhydride is added and the substance is stirred at room temperature until the anhydride dissolves. The peptide mixture is preserved by adding benzalkonium chloride in the amount of 0.04% of the volume of the drug. The mixture of peptides created is lyophilized in sterile beakers and further used for the study of its anti-viral activity and the confirmation of the mechanism of action.

**Example 9: Confirmation of Albuvir's Mechanism of Action**

Complexes of α-β-importins are universal proteins that transport viral polynucleotides (RNA or DNA) to the cell nucleus. It has a conservative amino acid identification peptide, which recognizes nuclear pore proteins and opens them so that the nucleoprotein and importin complexes can enter. To confirm the peptide's mechanism of action, we used DNA from the type 1 L-2 herpes viral strain. The DNA was separated out using a known method. The DNA was conjugated with particles of colloidal gold. The complex obtained was included in liposomes. Appropriately, the experiment was described with the SV40 virus in Old World monkeys in [[xxxiii]].

These liposomes merged with the cell membranes from the chicken fibroblast culture. After the merging of the liposome with the cell membrane, the virus's DNA entered the cytoplasm along with the gold particles. The cellular analog of the T-antigen carried the colloidal particles into the nuclear pores with the polynucleotide. If the cells were incubated with the group of peptides that are being patented, aggregation of the particles of colloidal gold in the nuclear pores was not observed. All the particles were equally distributed throughout the cells' cytoplasm. In this case, the cytopathic activity of the virus was not observed. Thus the drug designed slows the process of the transportation of viral DNA to the cell nucleus, which was to be proven.

**Example 10: Albuvir Treatment Effects on Mice Infected with Herpes Encephalitis**

Twenty BALB-C white mice were infected intraperitoneally with a lethal dose of 0.01 ml 7.01g HSV1, strain L2. After two days, the majority of the animals showed signs of encephalitis: sluggishness, reduction in food intake, and convulsions. The treatment was begun on the second day after infection. A group of modified peptides were administered perorally at a dosage of 10 mg/kg animal weight twice a day for five days. The infected animals in the control group were administered a 0.9% solution of sodium chloride. The main criterion for the effectiveness of the drug was the percentage of surviving animals in the main experimental group against the control group at 17 days. In the experimental group of animals that had been given the drug, only two out of 25 animals died (8%), while in the control group, 18 of 20 animals died (90%). In addition, on the second day after the treatment with the group of modified peptides was begun, the symptoms of herpes encephalitis had disappeared. Thus the drug had an obvious therapeutic effect in the treatment of herpes encephalitis in mice.

**Example 11: The Therapeutic Effect of the Drug on Models of Rabbits with Ophthalmic Herpes (OH)**

The most convenient method for the creation of OH and the evaluation of the effectiveness of drugs under these conditions is the models of herpes keratitis and keratoconjunctivitis in rabbits. The HSV-1 L2 strain was used in the form of a cultured fluid infected by a culture of Hep-2 cells. The maximun value of the infectious titer was 5.0-5.5 lg LD50/0.03 ml. Rabbits of both sexes with a weight of 2.5 kg were used. To create OH models, the rabbits' eyes were rinsed with a saline solution and anesthetized with Ultracaine injected into the conjunctiva. HSV-1 was applied to the abraded corneas at a infective dose (6-7 drops). The intensity of the clinical symptoms of the rabbits' OH was evaluated using a four-point system for each symptom, which were later tallied. The evaluation of the effectiveness of the chemical drugs was conducted taking into account the difference in symptoms displayed (Level of Intensively of Viral Symptomatic - LIVS in points) in the experimental and control groups (infected animals that were administered a 0.9% solution of sodium chloride and a group of animals that were administered the prototype drug). The LIVS in the control group (untreated animals) was taken to be 100%. The drug was administered 48 hours after infection at a dose of 0.01 g drug per rabbit three times a day perorally in a solution of distilled water. Analogously, but parenterally (in an ear vein), acylated albumin from the prototype and a 0.9% NaCl solution was administered. In 90% of the rabbits, the intensity of the conjunctivitis alone rated three to four points. The duration of the symptoms was an average of 21 days. They achieved their maximum development in four to eight days (LIVS at a level of eight to ten points). The evaluation criteria for the therapeutic effect of the drugs were: a) a lessening in the intensity of the clinical symptoms; b) a reduction in the length of the illness; c) prevention of lethal meningoencephalitis (survival). The data on the effectiveness of the drugs studied is presented in Table 11.

**Table 11: The Effect of Treatment of Infected Rabbits with Albuvir**

| Drug Name | Number of Animals | Length of Illness (Days) | LIVS (Points) |
|---|---|---|---|
| Albuvir | 7 | 7.5± 2.5* | 36.3 ± 4.8 |
| Parenteral Acylated Albumin | 7 | 25.5 ± 2.5* | 100± 2 |
| Untreated Control | 5 | 26 ± 2,5** | 109,2± 2,2 |
| * P <0.001 ** P<0.01 | | | |

As may be seen in the table, the use of Albuvir led to a halving of the symptoms of the illness in comparison to those in the animals that were infected but not treated. After its application, not only a lessening in the severity of symptoms, but also of the length of the illness by two thirds at a survival rate of 100% was observed. The LIVS for the drug were less than the LIVS for the control. At the same time, the acylated albumin from the prototype administered in the same dosage parenterally (intravenously in the ear vein) did not affect the life span or the length of the illness.

**Example 12: Research of the Anti-Influenza Properties of the Substance to Be Patented on *in ovo* Models**

Strains of the Influenza- A- Hong Kong- 95 H3N2 virus were used in the experiments. A total of sixty 10- 11- day- old chicken embryos were used in the experiment. Twenty embryos were used for each drug: 0.1 ml of the cultured fluid with 7 lg virus titer was introduced to the embryos' amniotic cavities; they were incubated for 1 day at a temperature of 35° C. Then 0.3 ml each of the experimental drug (from a calculated 0.003 g drug per kg weight) was introduced into the amniotic cavities of 20 of the infected embryos. 20 more embryos were administered 0.3 ml each of acylated albumin from the prototype, and 20 of the embryos were administered 0.3 ml each of a 0.9% NaCl solution. After three days, the embryos were dissected and a hemoagglutination reaction was initiated with a 5% suspension of erithrocytes. Allantoic and amniotic fluids were used as material in the hemoagglutination reaction. The virus titer was established through tenfold dilution in allankoic and amniotic fluids against a pure virus (Table 12) .

**Table 12: Anti-Influenza Activity of the Union Acylated Peptides on *in ovo* Models**

| Drug | Virus Titer * (lg) | | |
|---|---|---|---|
| | In Allantoic Fluid | In Amniotic Fluid | In Control (Mixture of Allantoic and Amniotic Fluids of Non-Infected Embryos) |
| Albuvir | 1.5±0.2 | 0 | 0 |

(continued)

| Drug | Virus Titer * (lg) | | |
|---|---|---|---|
| | In Allantoic Fluid | In Amniotic Fluid | In Control (Mixture of Allantoic and Amniotic Fluids of Non-Infected Embryos) |
| Acylated Albumin from the Prototype | 7.2±1.2 | 6.5±1.5 | 0 |
| 0.9% Saline Solution | 7.5±1.0 | 6.5±1.5 | 0 |
| * when P<0.001 | | | |

As can be seen in Table 12, the substance being patented reduces the concentration of the influenza virus by 6 lg, while the acylated albumen does not have anti-influenza properties. In the amniotic fluid into which the drug was introduced, the virus was not found at all. It is possible that, after ending up in the cell cytoplasm, it disintegrates under the influence of proteases and nuclease. This bears witness to another mechanism of the action of hydrolyzate of acylated albumin, in opposition to pure acylated albumin. The latter, as an inhibitor of virus adhesion, is capable only of protecting cells from being infected by viruses, but did not block the replication of the virus in infected cells.

**Example 13: Design of Vaccines for *Pseudomonas* Based on Self-Organizing Quasi-Life Systems**

In today's world, vaccinations are one of the main methods of preventing epidemics. There are two basic groups of infectious diseases: the group of infections controlled by vaccines (whose use prevents epidemics) that belong to the required vaccination scheme, and the second group of infections, against which vaccinations are of little or no effect [xxxiv] . Belonging to the first group of infections are conservative microorganisms and viruses whose antigen components are unchanging and a vaccine induces high levels of protective antibodies in the blood. These are infections such as diphtheria, pertussis, measles, rubella, etc. The second group of infections, against which vaccinations are ineffective, are those such as influenza, herpes infections, HIV/ AIDS, and certain others [xxxv,xxxvi,xxxvii] . There are many reasons for the ineffectiveness of this vaccine in preventing this group of infections. For example, the influenza virus is a polymorphic (the virus particle does not have a precise structure and form) virus with a fragmented, variable genome (Fig. 7.) .

The influenza virus is very changeable and persistent (lifetime residence in the human body) [xxxviii] . In the bodies of humans and animals [including birds [xxxix] ) this virus multiplies in several stages: in the intensely productive phase, the infected cell divides the viral particles, which are capable of infecting neighboring cells [xl] . In the persistence (latent) phase, this virus "waits around" inside the cell, meanwhile losing a part of its fragmented genome or picking up particles of human RNA in the cytoplasm [xli] . According to statistics, a virus's antigen content changes by 5% per month [xlii] . Accordingly, the application of standard approaches to the development of flu vaccines is not promising. Even the application of recombinant proteins and new types of gene vaccines does not prevent these drugs from aging quickly. The presence in one ampoule of several conservative proteins (for example, hemoagglutinins and neuroamidinases for the flu virus) does not permit the protection of the organism from viral aggression through inducing the production of specific antibodies. These antibodies will not have the same monoclonal specificity at all, which will be necessary at this level of virus mutation. The change in the approach to vaccine design must be accompanied by the inclusion of antigens in the vaccine that have not yet appeared as the result of viral mutation [xliii] . The so- called predicative inclusion of antigens is possible through two methods: the classical, with the application of methods for the epidemiological prediction of antigenic drift, or the method of the partial modification of antigens with the obtainment of an unlimited quantity of antigen combinations in one ampoule of antigen. The first approach has only partially proven itself: in no case has the antigenic drift prediction coincided with the real mutational changes to flu neuroamidinases and hemoagglutinins [xliv,xlv] . If we use the technique of partial modification of the protein component of the vaccine antigen- for example, type 1 neuroamidinases- in the vaccine preparation process, then in one dose of the vaccine, in place of one protein with one antigen profile, more than a million proteins with one primary and secondary structure, but with differing replacement sites and antigen profiles will appear.

The antibodies induced by this protein will cover all possible attachment site combinations. Accordingly, the quantity of induced monoclones will be of an order higher, although the protein will remain the same. Also, any "future" epitope of the structure of a neuroamidinase will be covered by antibodies that have already been synthesized.

This approach permits us to sharply limit the quantity of antigen for vaccination, to protect the organism from viruses with a fragmented genone and highly changeable microorganisms using a small quantity of antibodies but with a significantly larger spectrum of monoclonal specificity. Roughly speaking, the vaccine will contain a selection of even those neuroamidinase antigens that do not even exist yet. Additionally, the blood of animals vaccinated earlier will already

contain a necessary pool of antibodies to "future" viral strains. The use of this vaccine will allow us to successfully protect an organism from highly changeable and persistent, as well as low-immunogenic, viruses and microorganisms.

*Pseudomonas aeruginosa* (PMA) was cultivated on a solid medium (plain broth with 1% glucose added). After three days, the surface of the medium was completely covered with *Pseudomonas aeruginosa.* From the surface of the medium in the Petri dish, a lavage was done with a 0.9% solution of sodium chloride; the suspension obtained was rinsed and centrifuged three times. After a repeat suspending of the suspension, the microorganisms were heated in a thermostat over 140 minutes at a temperature of 80° C and then were again inoculated against PMA for the purpose of inactivation control. In quantity of microbial bodies, the suspension obtained measured at 10 billion cells/ml; then 0.1 ml of the suspension was diluted 100 times with a 0.9% solution of sodium chloride, and the concentration of surface proteins was determined using the biuret method and in complexing reaction with the blue phenyl bromide Flores method [3]. In conversion to proteins, an acylation reaction was conducted with a succinic anhydride solution as indicated in Example 1 [6]. Eight samples were obtained with varying levels of acylation: 1%, 3%, 5%, 7%, 9%, 11%, 13 %, and 15%. Exceeding 15% completely deprives the succinylated proteins of immunological potency; therefore, it was not considered worthwhile to obtain and use those produced with a modification level of more than 15% in future. The corpuscular antigen with a different level of acylation was used further for determination of its immunological potency. Another part of the antigen was centrifuged for 40 minutes at 3 000 revolutions per minute. The sediment was removed, and the supernatant was run through a column of Sephadex G-75. The first, heaviest fraction was collected and used further for establishing the concentration of protein and the level of chemical modification. The antigen obtained was a homogeneous fraction (one polymeric substance) and had a molecular weight of 1.5 mDa and a charge of - 186000. A diluted glycoprotein antigen was obtained with these levels of acylation: 1%, 3%, 5%, 7%, 9%, 11%, 13 %, 15%.

It is well known that when gel filtration is used, the separation of proteins takes place according to the sizes of the protein globules. Gel filtration [7] was conducted in columns filled with Sephadex G-75 gel. The total volume of the column of gel was determined to be Vt. Throughout the elution, the large molecules that do not penetrate the gel granules move quickly in combination with the intergranular solution and appear in the form of a narrow layer. The volume of eluent that corresponds to the appearance of this zone was determined to be $V_0$ (free volume). The smaller molecules passed through the column slowly and penetrated the granules of the gel, and their exit from the column took quite a long time. In connection with the fact that the level of diffusion in the gel granules depends on the size of the molecules, the substances were removed from the column in decreasing order of their molecular mass. The molecular weight of a M of the experimental protein was established through comparison of a volume of eluted Ve with the analagous parameters of the protein markers.

To separate out the antigen, a column with a diameter of 25 mm and a length of 1000 mm was used. The G-75 and G-25 Selphadex gel was prepared ahead of time thus: gel granules were slowly added to a buffer solution (0.1 M tris -HCl and 0.1 M NaCl, pH=8.0); this was kept in a thermostat for 72 hours at a temperature of 37 degrees C. Then the gel was deaerated (gradually and carefully mixed to an excess amount of buffer solution to remove gas bubbles) in a shaker over the course of an hour. Filtration paper was placed in the bottom of the column. A small amount of buffer solution was slowly added to the column; then a small quantity (5 g) of the suspension of swollen Selphadex G-25 gel granules was added along the wall of the container. After the formation of the gel column, no less than two volumes of reaction buffer were passed through the column. Then 100 mcl of the sample solution was extracted using a micropipette. The constant speed of the elution was set through installing a drip over the column with a buffering solution and regulating the speed of the elution using a rolling regulator. The eluent was collected in 0.5 ml test tubes and analyzed with an SF-56 spectrophotometer at a wavelength of 280 nm using method [4].

The anti-*Pseudomonas* serum for diagnostic purposes with a titer of specific anti-*Pseudomonas* antibodies (1: 1000) was obtained through a standard mouse immunization scheme, according to scheme [1] of the thermally inactivate corpuscular *Pseudomonas* vaccine with a particle concentration of 10 billion/ml, which was administered at 3; 5 and 7 days intramuscularly at a dose of 0.2 ml. Twenty mice were used to obtain reference serum.

To immunize the animals in the experiment, acylated samples of both the corpuscular antigen (ten animals per sample, eight groups) and the acylated antigen in solution (eight samples, ten animals per sample) were taken. The first group of animals were administered 8 samples of antigen (10 animals per sample perorally at 0.2 ml each) according to the Pasteur scheme (at 1; 3 and 7 days); the second group was administered samples in accordance with the scheme developed by Prof. E. M. Babich (every other day, 0.2 ml perorally over 15 days) [1]. The level of antibodies was established through two methods: hemoagglutinin reaction and antibody fluorescing. Three animals from each group were left alive until the 15-day mark, then killed with ether and used to obtain serum, in which the level of specific antibodies was also established using the abovementioned methods. For the implementation of the abovementioned, a test system was prepared for a direct hemoagglutination reaction, which was conducted in round-bottomed 96-well immunology plates, to which was added 0.02 ml each of a 0.1% suspension of thermostatted sheep erythrocytes and 0.02 ml each of a suspension of thermally inactivated *Pseudomonas* cells in a concentration of 10 billion cells/ml. The level of antibodies was established in sequenced tenfold (but twofold) mouse blood serum cultures, which were added to microtiter wells in the amount of 0.02 ml. The presence of agglutinates bore witness to the creation of immune

complexes. Normal human immunoglobins (a titer of anti-*Pseudomonas* antibodies made up from 0 to [1:10] in accordance with AND) and a serum of the blood of unvaccinated mice (titer from 0 to 1:10) were used as controls.

The first sample was a model of a corpuscular vaccine based on inactivated pasteurization of *Pseudomonas aeruginosa.* Only the surface antigens were acylated. The level of acylation varied from 1% to 15% in increments of 2%. In total, eight samples of modified antigen in solution and eight corpuscular antigens were studied. The result of the study of the immunogenic potential of the samples obtained is illustrated in the data of Fig. 8.

As may be seen in Fig. 8, 15 days after the injection of the native vaccine drug, the average titer of antibodies in the vaccinated animals came to (1:640). The peroral use of the native corpuscular antigen did not cause induction of the synthesis of specific antibodies.

The chemically modified antigen from the modification stage at 1% of the concentration of protein in it induced the synthesis of the same level of antibodies as did the native antigen (1:640). Modification of surface antigens by 3% led to the induction of antibodies on the level (1:320) for the peroral variation and on the level of (1:2560) for the injected variation.

For the derivative antigens with other levels of acylation, induction of the synthesis of specific anti-*Pseudomonas* antibodies was not observed when they were administered perorally. The injection variation was effective even in derivatives with a 15% level of acylation. The derivative with an acylation level of 5% induced antibody synthesis at a level of (1:1280); the derivative with a 7% level of acylated antigen did so at a level of (1:320); the 9% derivative had a level of (1:40), and other variations (from 11-15% modified) had a level of (1:20).

Thus the most effective derivative turned out to be the one with the 3% acylation level, which induced the synthesis of specific antibodies both when the classic Pasteur vaccination scheme was used with injection and when the E. Babich peroral vaccination scheme was used.

In Fig. 9, the dependence is shown between the level of initiated specific antibodies and the level of acylation of the modified antigen in solution (first fraction).

This antibody-candidate variant is a chemical monocomponent vaccine based on a modified high-molecular glycoprotein antigen with a mass of 1.5 mDa and a molecular charge of 186000. It is the higher molecular weight or the first fraction that leaves the column upon division of the fraction that has turned out to be the most immunologically potent. In the study of the structure of the antigen obtained, the correlation between the change in charge and the mass of the antigen was confirmed, which in turn confirms the condition of the cessation of the chemical reaction of antigen structural modification. An interesting fact discovered during the injection vaccination of the mice should be noted: administering the unmodified vaccine (on the seventh day, the third time) caused an allergic reaction in some of the animals in the form of tremors, a fall in motor activity, and a refusal of food over the course of one day.

The introduction of all of the modified variants of the vaccine did not cause side effects in animals. The acylated variant of the antigen in solution with a 1% level of acylation (Fig. 9), like the non-acylated antigen, caused the induction of the synthesis of an identical quantity of antibodies in titer (1:1280). The peroral administration of the antigen in solution and the antigen with an acylation level of 1% did not lead to a substantial induction of the synthesis of specific anti-*Pseudomonas* antibodies. The derivative of the antigen in solution with an acylation level of 3% activated the synthesis of antibodies at a level of (1:5120) in the injected form and (1:1280) in peroral use. The 5% acylated derivative induced the synthesis of specific antibodies at a level of (1:640) for the peroral form of administration and at a level of (1:2560) for the injected form. The 7% acylated derivative induced the synthesis of specific antibodies at a level of (1:640) for the peroral form of administration and at a level of (1:1280) for the injected form. The 11% acylated derivative correspondingly induced the synthesis of specific antibodies at a level of (1:320) for the peroral form of administration and at a level of (1:640) for the injected form. The level of antibody synthesis was equal in vaccinated animals in the peroral and injection administrations only when the vaccine drug with an acylation level of 13% was used, coming to 1:40; at the 15% acylation level, only the injected form of the vaccine candidate was immunogenic: it induced antibody synthesis at a level of (1:20).

Thus the characteristic distinction of the high-molecular antigen in solution turned out to be the induction of antibody synthesis not only by derivatives with a 3% level of acylation, but by derivatives with a 5%, 7%, 11%, and 13% acylation level in peroral administration in the abovementioned vaccination scheme as described by E. M. Babich. The antibody titer of the most effective derivative with a 3% acylation level was four times more effective in injection form than it was in peroral form. In comparison with the corpuscular modified antigen, the 3% acylated derivative of the antigen in solution was twice as immunogenically effective in injection form and four times more effective in peroral form. Despite an antigen load that is significantly higher than in the injected form, the peroral administration of partially acylated antigen in solution turned out to be effective, and induced a level of antibodies that has a perspective of protecting an organism from *Pseudomonas* infection in the long term (a year or more). The use of a peroral vaccine is without a doubt a promising direction for the improvement of immunobiological substances.

Among the variations of acylated antigens, the glycoprotein antigen in solution succinic with 3% of the mass of the protein was chosen, which with a peroral administration for 15 days caused the induction of the synthesis of specific antibodies in a titer of (1:1280) and to a titer of (1:5120) in injection form.

## Industrial Applicability

This invention is related to medicine and pharmaceuticals, and may be used for the design and creation of new, less expensive and more effective drugs that will be self-adapting and self-organizing systems. The drugs obtained in this manner are completely ecologically safe, biodegradable, and fully metabolized both in patients' bodies and in the environment; the technology required to make them is completely without waste, and its production and design costs are hundreds of times smaller than their existing counterparts on the existing equipment of pharmaceutical companies and laboratories.

## References [i]

□. Lundstrom K. Structural genomics and drug discovery. J Cell Mol Med. 2007 Mar- Apr; 11 (2) : 224- 38.

[ii] . Ma B, Nussinov R. Trp/Met/Phe hot spots in protein- protein interactions: potential targets in drug design. Curr Top Med Chem. 2007; 7 (10) : 1007- 13. Links

[iii] . Keskin O, Gursoy A, Ma B, Nussinov R. Towards drugs targeting multiple proteins in a systems biology approach. Curr Top Med Chem. 2007; 7 (10) : 945- 53.

[iv] Shchekotikhin A.E., Dezhenkova L.G., Susova O.Y., Glazunova V.A., Luzikov Y.N., Sinkevich Y.B., Buyanov V.N., Shtil A.A., Preobrazirenskaya M.N. Naphthoindole-based analogues of tryptophan and tryptamine: synthesis and cytotoxic properties.// Bioorg. Med. Chem.- 2007.- V.15.- P.2651-2659.

[v] Patent No.: US 5,495,423 General strategy for vaccine and drug design [vi] Patent No.: US 6,226,603 Bl Method for the prediction of binding targets and the design of ligands

[vii] Patent No.: US 5,854,992 System and method for structure-based drug design that includes accurate prediction of binding free energy

[viii] Patent N: US 6,226,603 B1 Method for the prediction of binding targets and the design of ligands

[ix] Patent N: US 5,854,992 System and method for structure-based drug design that includes accurate prediction of binding free energy

[x] http://dic.academic.ru/dic.nsf/ruwiki/79240

[xi] Jean-Marie Lehn. Supramolecular Chemistry. Concepts and Perspectives.- Weinheim; New York; Basel; Cambridge; Tokyo: VCH Verlagsgesellschaft mbH, 1995.-P. 103 (Chapter 7)

[xii] . Butcher LA, Tomkins JK. A comparison of silver staining methods for detecting proteins in ultrathin polyacrylamide gels on support film after isoelectric focusing. Anal Biochem 1985; 1: 384-8.

[xiii] Melen K, Ronni T, Broni B, Krug RM, von Bonsdorff CH, Julkunen I. Interferon-induced Mx proteins form oligomers and contain a putative leucine zipper. J Biol Chem. 1992267, 25898-907.

[xiv] www.ncbi.nlm.nih.gov/entrez/query.fcgi?db=Structure? 1RH2

[xv] Galasso M, Elena Sana M, Volinia S. Non-coding RNAs: a key to future personalized molecular therapy?// Genome Med. 2010 Feb 18;2(2):12.

[xvi] Jung J, Solanki A, Memoli KA, Kamei K, Kim H, Drahl MA, Williams LJ, Tseng HR, Lee K. Selective inhibition of human brain tumor cells through multifunctional quantum- dot- based siRNA delivery// Angew Chem Int Ed Engl. 2010; 49 (1) : 103- 7.

[xvii] Li X, Liu Y, Wen Z, Li C, Lu H, Tian M, Jin K, Sun L, Gao P, Yang E, Xu X, Kan S, Wang Z, Wang Y, Jin N.Potent anti-tumor effects of a dual specific oncolytic adenovirus expressing apoptin in vitro and in vivo// Mol Cancer. 2010 Jan 20;9:10.

[xviii] Emmrich S, Wang W, John K, Li W, Pützer BM. Antisense gapmers selectively suppress individual oncogenic p73 splice isoforms and inhibit tumor growth in vivo// Mol Cancer. 2009 Aug 11;8:61.

[xix] Fisher M, Abramov M, Van Aerschot A, Rozenski J, Dixit V, Juliano RL, Herdewijn P. Biological effects of hexitol and altritol- modified siRNAs targeting B- Raf// Eur J Pharmacol. 2009 Mar 15; 606 (1- 3) : 38- 44

[xx] Czauderna F, Fechtner M, Dames S, Aygün H, Klippel A, Pronk GJ, Giese K, Kaufmann J. Structural variations and stabilising modifications of synthetic siRNAs in mammalian cells// Nucleic Acids Res. 2003 Jun 1; 31 (11) : 2705- 16.

[xxi] Crooke RM, Graham MJ, Martin MJ, Lemonidis KM, Wyrzykiewiecz T, Cummins LL. Metabolism of antisense oligonucleotides in rat liver homogenates. J Pharmacol Exp Ther. 2000 Jan; 292 (1) : 140- 9.

[xxii] Monia BP, Johnston JF, Sasmor H, Cummins LL.Nuclease resistance and antisense activity of modified oligonucleotides targeted to Ha- ras. J Biol Chem. 1996 Jun 14; 271 (24) : 14533- 40.

[xxiii] Giles RV, Spiller DG, Green JA, Clark RE, Tidd DM. Optimization of antisense oligodeoxynucleotide structure for targeting ber- abl mRNA. Blood. 1995 Jul 15; 86 (2) : 744- 54.

[xxiv] Xodo L, Alunni- Fabbroni M, Manzini G, Quadrifoglio F. Pyrimidine phosphorothioate oligonucleotides form triple-stranded helices and promote transcription inhibition. Nucleic Acids Res. 1994 Aug 25; 22 (16) : 3322- 30.

[xxv] Sazani P, Weller DL, Shrewsbury SB. Safety pharmacology and genotoxicity evaluation of AVI- 4658.Int J Toxicol. 2010 Mar- Apr; 29 (2) : 143- 56.

[xxvi] Mourich DV, Jendrzejewski JL, Marshall NB, Hinrichs DJ, Iversen PL, Brand RM. Antisense targeting of cFLIP sensitizes activated T cells to undergo apoptosis and desensitizes responses to contact dermatitis. J Invest Dermatol. 2009 Aug; 129 (8) : 1945- 53.

[xxvii] Belon CA, Frick DN. Helicase inhibitors as specifically targeted antiviral therapy for hepatitis C. Future Virol. 2009 May 1; 4 (3) : 277- 293

[xxviii] http://www.agrovet.com.ua/index.php?id=25

[xxix] Bagnyukoval T.V., Pogribny I.P. Chekhun V.F. MicroRNAs in normal and cancer cells: A New class of gene expression regulators// Experimental Oncology.-2006, N 28.- P. 263-269

[xxx] Steinhauser I, Langer K, Strebhardt K, Spänkuch B. Uptake of plasmid- loaded nanoparticles in breast cancer cells and effect on Plk1 expression. J Drug Target. 2009 Sep; 17 (8) : 627- 37. [xxxi] Lu JJ, Langer R, Chen J.A novel mechanism is involved in cationic lipid- mediated functional siRNA delivery. Mol Pharm. 2009 May- Jun; 6 (3) : 763- 71.

[xxxii] Moreira JN, Santos A, Moura V, Pedroso de Lima MC, Simões S. Non- viral lipid- based nanoparticles for targeted cancer systemic gene silencing. J Nanosci Nanotechnol. 2008 May; 8 (5) : 2187- 204.

[xxxiii] Lanford R.E., Butel J.S. Cell.- 1984.Vol.37., P. 801-813

[xxxiv] .Robbins, J. B., R. Schneerson, and S. C. Szu. 1995. Perspective: hypothesis: serum IgG antibody is sufficient to confer protection against infectious disease by inactivating the inoculum. J. Infect. Dis. 171:1378-1398.

[xxxv] . Del Val, M., H. J. Schlicht, H. Volkmer, M. Messerle, M. J. Reddehase, and U. H. Koszinowski. 1991. Protection against lethal cytomegalovirus infection by a recombinant vaccine containing a single nonameric T-cell epitope. J. Virol. 65:3641-3646

[xxxvi] . Larsen, D. L., A. Karasin, and C. W. Olsen. 2001. Immunization of pigs against influenza virus infection by DNA vaccine priming followed by killed-virus vaccine boosting. Vaccine 19:2842-2853

[xxxvii] . Cicin-Sain, L., Brune, W., Bubic, I., Jonjic, S., Koszinowski, U. H. (2003). Vaccination of Mice with Bacteria Carrying a Cloned Herpes Virus Genome Reconstituted In Vivo. J. Virol. 77: 8249-8255

[xxxviii] . Levin SA, Dushoff J, Plotkin JB. Evolution and persistence of influenza A and other diseases. Math Biosci. 2004 Mar-Apr;188:17-28.

[xxxix] . Terregino C, Toffan A, Beato MS, De Nardi R, Drago A, Capua I. Conventional H5N9 vaccine suppresses shedding in specific-pathogen-free birds challenged with HPAI H5N1 A/chicken/Yamaguchi/7/2004. Avian Dis. 2007 Mar;51(1 Suppl):495-7.

[xl] . Medvedeva MN, Petrov NA, Vasilenko SK, Simanovskaia VK, Golubev DB. The characteristics of the hemagglutinin from persistent variants of the influenza virus A/ Victoria/ 35/72 (H3N2) . Vopr Virusol. 1990 Sep- Oct; 35 (5) : 374- 6.

[xli] . Aronsson F, Robertson B, Ljunggren HG, Kristensson K. Invasion and persistence of the neuroadapted influenza virus A/WSN/ 33 in the mouse olfactory system. Viral Immunol. 2003; 16 (3) : 415- 23.

[xlii] . Cox MM. Vaccines in development against avian influenza. Minerva Med. 2007 Apr;98(2): 145-53.

[xliii] . Gronvall GK, Borio LL. Removing barriers to global pandemic influenza vaccination. Biosecur Bioterror. 2006; 4(2): 168-75.

[xliv] . Taubenberger JK, Morens DM, Fauci AS. The next influenza pandemic: can it be predicted? JAMA. 2007 May 9; 297 (18) : 2025- 7.

[xlv] . Vardavas R, Breban R, Blower S. Can influenza epidemics be prevented by voluntary vaccination? PLoS Comput Biol. 2007 May 4;3(5):e85.

## Claims

1. A method of molecular design and synthesis of therapeutic and preventive drugs in which a biopolymer target is used, including the determination of a target for the drug; the study, with the help of methods of molecular computer modeling, of the interactions are studied between the target and several models of drug ligands and the synthesis of the most active drug possible, dis**tinguished by the fact :** that in the capacity of a ligand, the same biopolymer target is used, which is cut into oligomer fragments; the fragments are modified through changing their charges to the opposite charge with the creation of supramolecular assemblies that are used as an active drug.

2. The method as per pt. 1, with the difference that protein is used as the biopolymer target.

3. The method as per pt. 1, with the difference that a mixture of proteins is used as the biopolymer target.

**4.** The method as per pt. 1, with the difference that milk is used as the biopolymer target.

**5.** The method as per pt. 1, with the difference that egg white is used as the biopolymer target.

**6.** The method as per pt. 1, with the difference that DNA is used as the biopolymer target.

**7.** The method as per pt. 1, with the difference that RNA is used as the biopolymer target.

**8.** The method as per pt. 1, with the difference that a mixture of DNA and RNA proteins and their combinations is used as the biopolymer target.

**9.** The method as per pt. 1, with the difference that the biopolymer target is cut into fragments using proteases.

**10.** The method as per pt. 1, with the difference that the biopolymer target is cut into fragments using nucleases.

**11.** The method as per pt. 1, with the difference that the biopolymer target is cut into fragments using synthetic nucleases.

**12.** The method as per pt. 1, with the difference that the charges of oligomer fragments of the biopolymer target are changed to their opposites through acylation.

**13.** The method as per pt. 9, with the difference that trypsin is used in the capacity of a protease.

**14.** The method as per pt. 1, with the difference that a partial change of the biopolymer target charge to its opposite is effected through acylation.

**15.** The method as per pt. 1, with the difference that a partial change of the biopolymer target charge to its opposite is effected through acylation.

**16.** The method as per pt. 1, with the difference that from 0.5 to 100% of the biopolymer target charge is changed to its opposite.

**17.** The method as per pts. 1, with the difference that acylation is caused by anhydrides of carboxylic and polycarboxylic acids.

**18.** The method as per pts. 1, with the difference that acylation is caused by halogen-producing carboxylic and polycarboxylic acids.

**19.** A method of molecular design and synthesis of therapeutic and preventive drugs that includes determination of the target of the drug's action; with the help of methods of molecular computer modeling, the interactions are studied between the target and several models of drug ligands and the synthesis of the most active drug possible, distinguished by the fact that the same biopolymer target is used, which is modified by partially changing the charge of the molecule to the opposite sign with the creation of supramolecular assemblies that are used as an active drug.

**20.** The method as per pt. 19, with the difference that protein is used as the biopolymer target.

**21.** The method as per pt. 19, with the difference that a mixture of proteins is used as the biopolymer target.

**22.** The method as per pt. 19, with the difference that milk is used as the biopolymer target.

**23.** The method as per pt. 19, with the difference that egg white is used as the biopolymer target.

**24.** The method as per pt. 19, with the difference that DNA is used as the biopolymer target.

**25.** The method as per pt. 19, with the difference that RNA is used as the biopolymer target.

**26.** The method as per pt. 19, with the difference that a mixture of DNA and RNA proteins and their combinations is used as the biopolymer target,

**27.** The method as per pt. 19, with the difference that the biopolymer target is cut into fragments using proteases.

**28.** The method as per pt. 19, with the difference that the biopolymer target is cut into fragments using nucleases.

**29.** The method as per pt. 19, with the difference that the biopolymer target is cut into fragments using synthetic nucleases.

**30.** The method as per pt. 19, with the difference that the charges of oligomer fragments of the biopolymer target are changed to their opposites through acylation.

**31.** The method as per pt. 27, with the difference that trypsin is used in the capacity of a protease.

**32.** The method as per pt. 19, with the difference that a partial change of the biopolymer target charge to its opposite is effected through acylation.

**33.** The method as per pt. 19, with the difference that a partial change of the biopolymer target charge to its opposite is effected through acylation.

**34.** The method as per pt. 19, with the difference that from 0.5 to 100% of the biopolymer target charge is changed to its opposite.

**35.** The method as per pt. 19, with the difference that acylation is caused by anhydrides of carboxylic and polycarboxylic acids.

**36.** The method as per pt. 19, with the difference that acylation is caused by halogen-producing carboxylic and polycarboxylic acids.

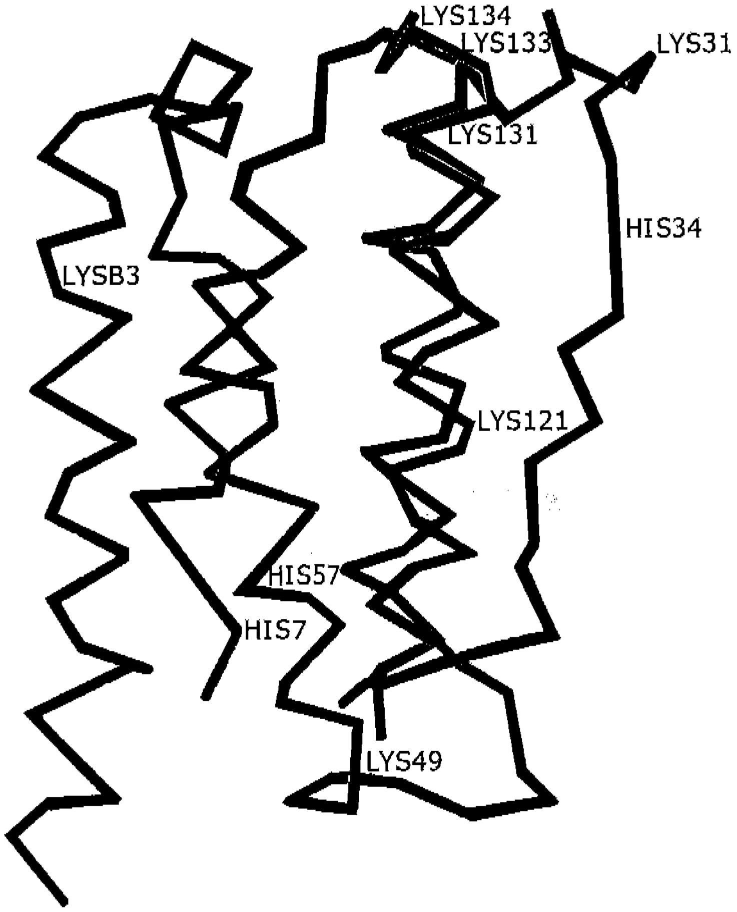


Fig. 1: Structure of α-2b-Interferon according to Data from the X-Ray-Structural Analysis (online public database of the US National Library of Medicine, www.ncbi.nlm.nih.gov/entrez/query.fcgi?db=Structure? 1RH2). Lysine, histidine, and arginine residues are depicted.

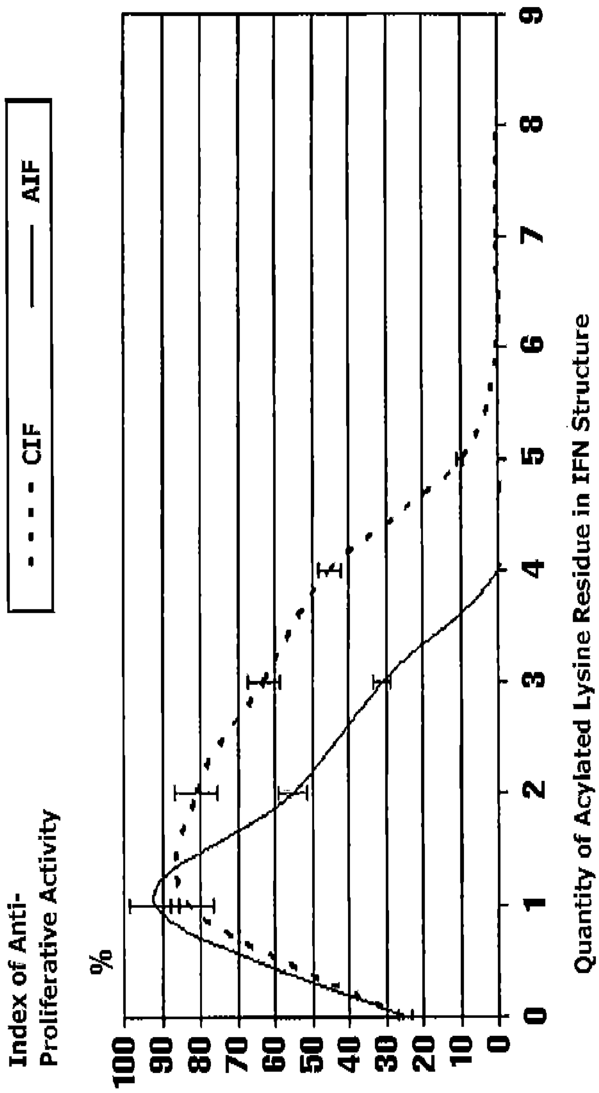


Fig. 2: Index of Anti-Proliferative Activity of IFN Derivatives toward CaOv Cells

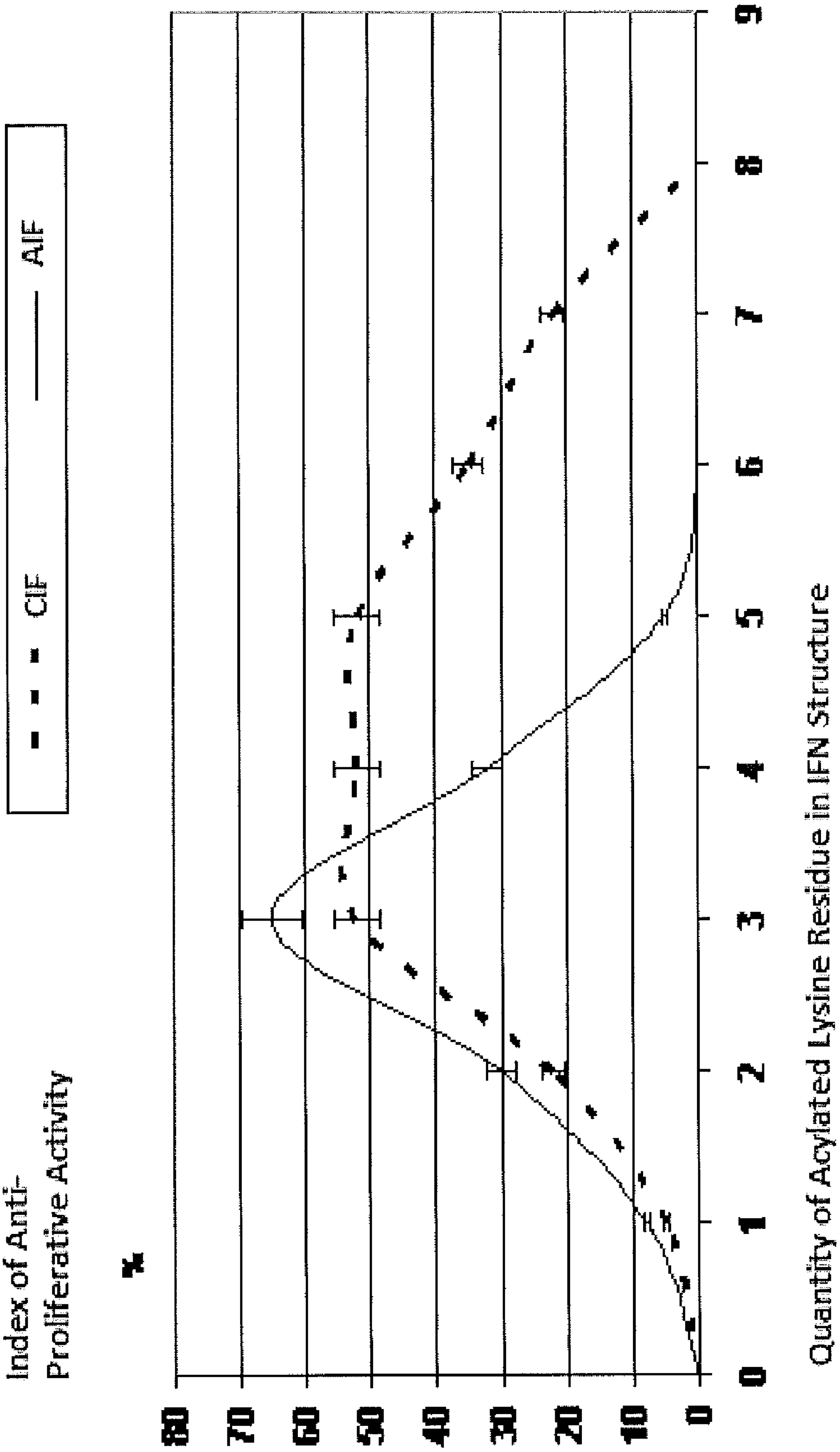


Fig. 3: Index of Anti-Proliferative Activity of IFN Derivatives toward SW-480 Cells

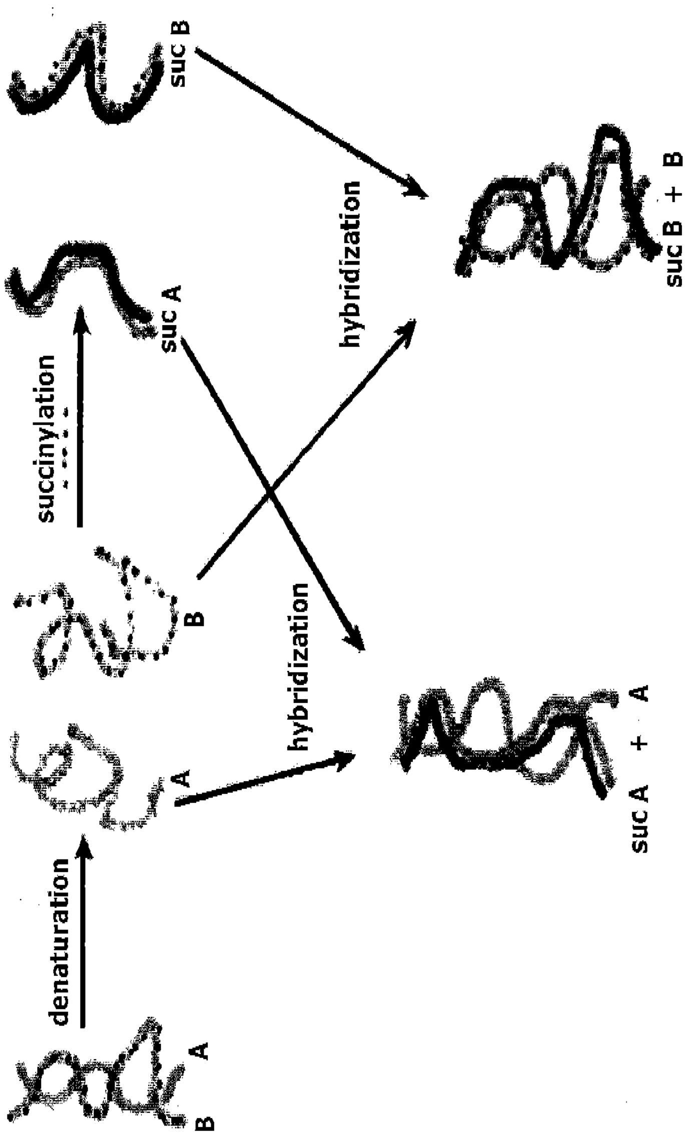


Fig. 4 (two sheets)

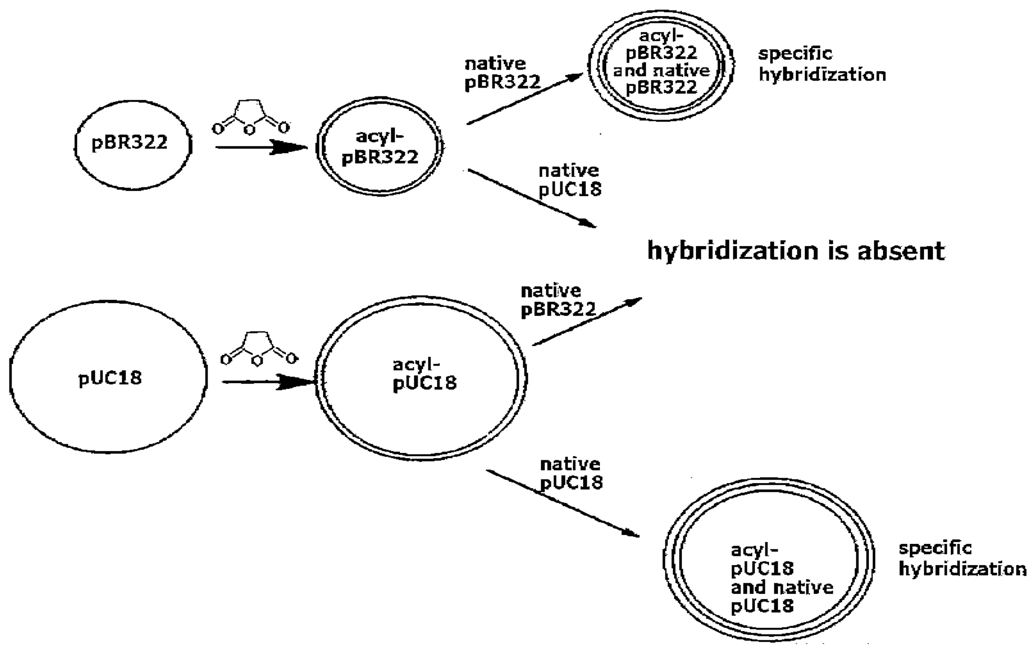


Fig. 4: Specific Hybridization of Acylated RNA Only with their Precursors (below: the same reaction with DNA plasmids

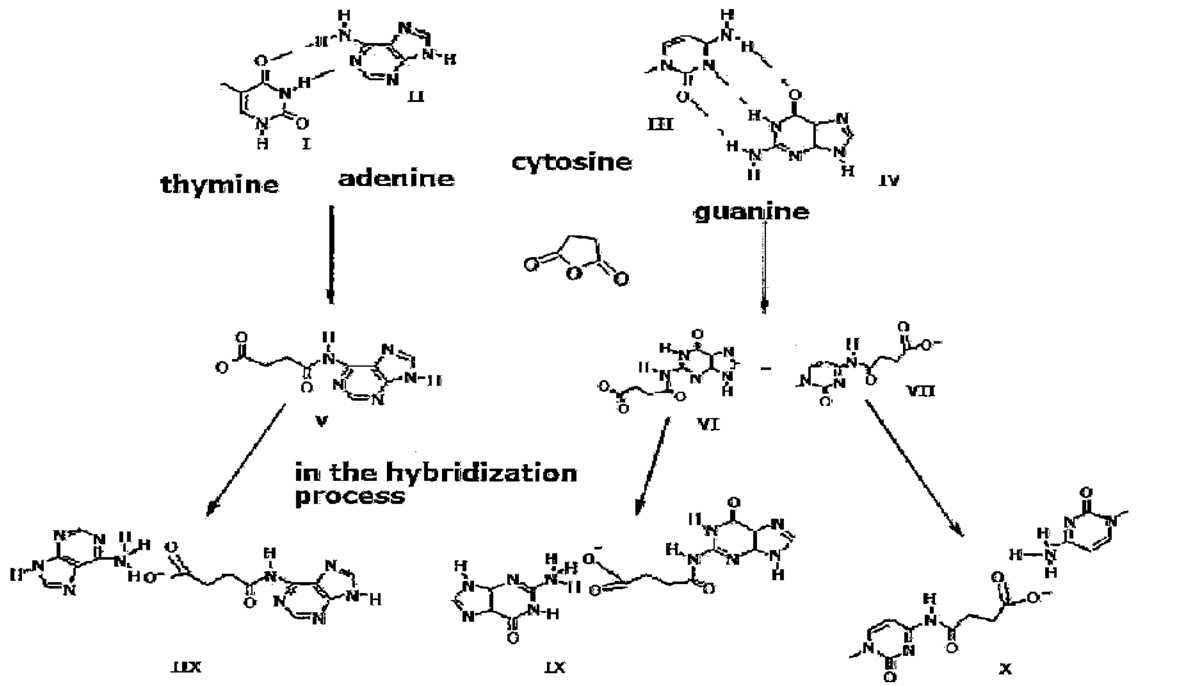


Fig. 5: Replacement of a Hydrogen Bond with an Ionic Bond in the Creation of Hybrids between Anticanum and the Target

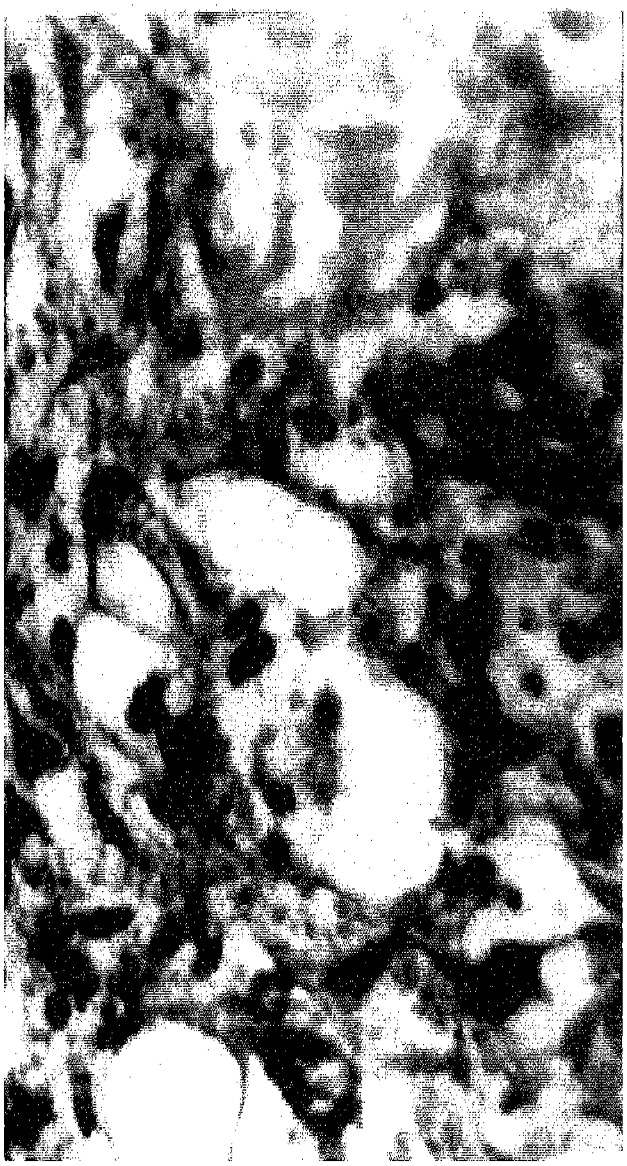

Fig. 6: Sarcoma. Hematoxylin-Eosin Dye

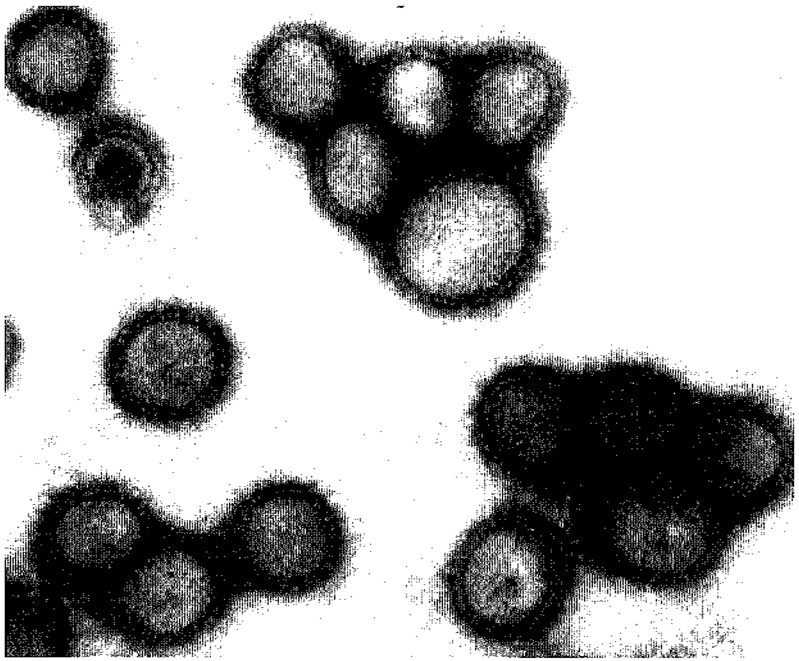

Fig. 7: Electronic Microphotography of the Influenza Virus (from www.news.wisc.edu/ newsphotos/influenza.html). The virus's polymorphism can clearly be seen in the virions of various sizes and shapes.

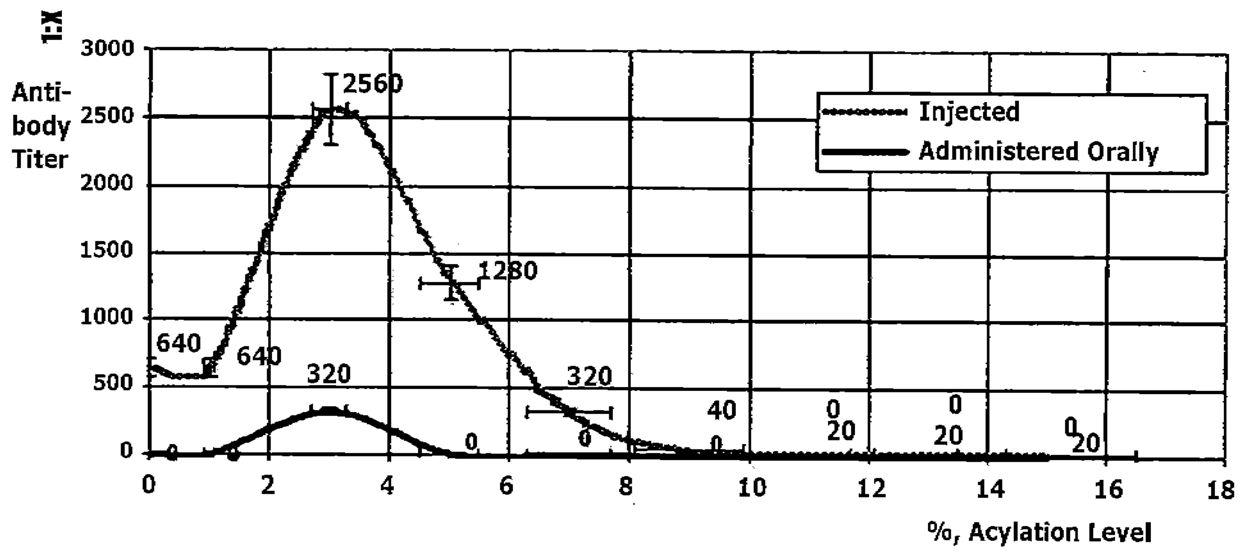


Fig. 8: Dependence between a Titer of Induced Specific Antibodies and the Acylation Level of a Corpuscular *Pseudomonas* Antigen

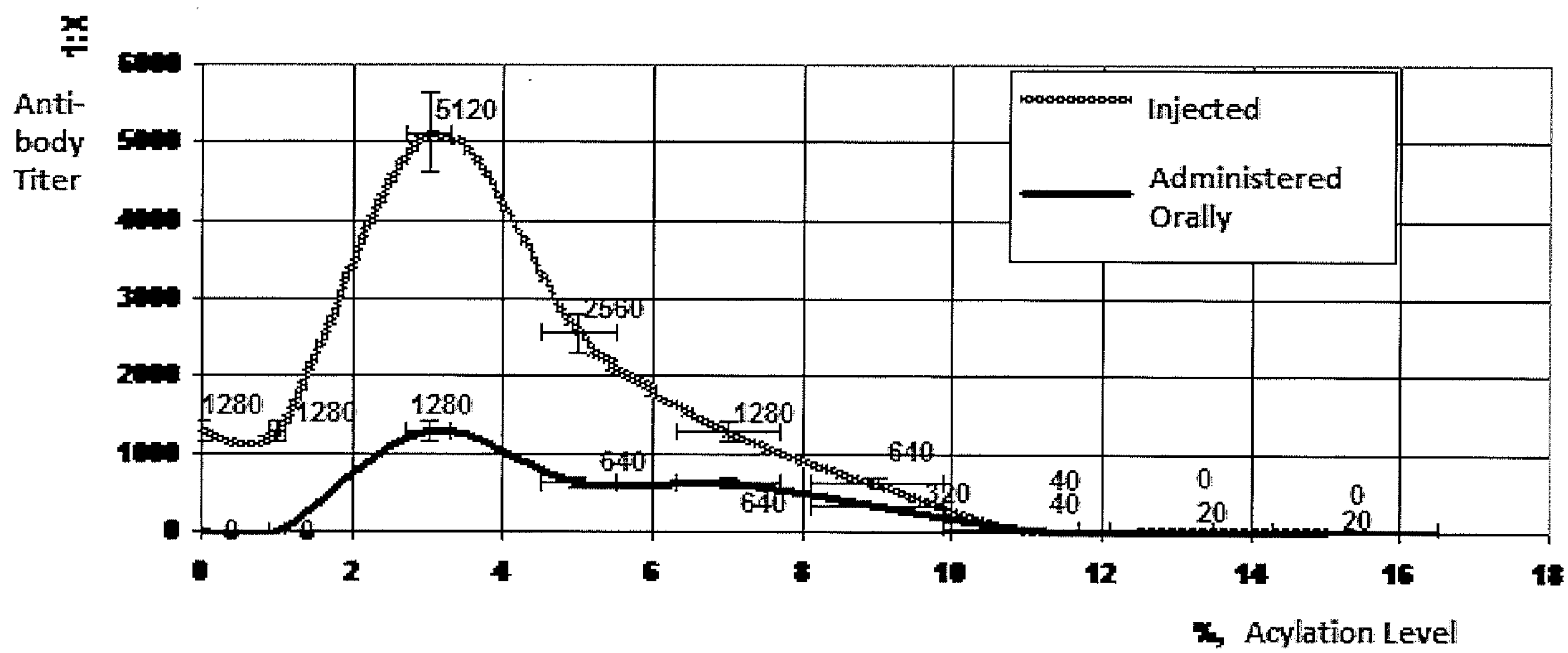


Fig. 9: Dependence between a Titer of Induced Specific Antibodies and the Acylation Level of a Soluble High-Molecular-Weight *Pseudomonas* Antigen

**INTERNATIONAL SEARCH REPORT**

International application No.
PCT/RU 2010/000694

A. CLASSIFICATION OF SUBJECT MATTER
C07K 1/06 (2006.01), C07H 21/00 (2006.01), G06F 19/18 (2011.01), C07B47/00

According to International Patent Classification (IPC) or to both national classification and IPC

B. FIELDS SEARCHED

Minimum documentation searched (classification system followed by classification symbols)
C07K 1/06, C07H 21/00, G06F 19/18, A61K 3 1/7088, 38/00, C07B 43/00, 43/06, 47/00, G09B 23/28

Documentation searched other than minimum documentation to the extent that such documents are included in the fields searched

Electronic data base consulted during the international search (name of data base and, where practicable, search terms used)
Esp@cenet, NCBI, PAJ, USPTO DB, WIPO, RUPTO, EAPATIS

C. DOCUMENTS CONSIDERED TO BE RELEVANT

| Category* | Citation of document, with indication, where appropriate, of the relevant passages | Relevant to claim No. |
|---|---|---|
| X<br>Y | Martynov A. V. et al. New approach to design and synthesis of therapeutic and preventive drugs, taking into account interspecies polymorphism of receptors (metod of precision partial modification). Annals of Mechnicov Institute, N°4, 2007, pages 5-13 | 1-3,6-8,12,14,16, 17,19-21,24-26,30,32,34,35<br>4-5,9-11,13,15,18,22,23<br>27-29,31,33,36 |
| Y | Masaaki Iigo et al. Orally administered bovine lactoferrin induces caspase-1 and interleukin-18 in the mouse intestinal mucosa: a possible explanation for inhibition of carcinogenesis an metastasis. Cytokine, 2004, 25: 26-44, especially, the abstract, page 42, paragraph 4, 1, discussion | 4,9,22,27 |
| Y | Francis R. Carbone et al. Induction of ovalbumin-specific cytotoxic T cell by in vivo pertide immunization. J. Exp. Med., 1989, vol. 169: 603-612, especially, page 605, lines 8-20, figure 1, discussion | 5,9,13,23,27,31 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 June 2011 (21.06.2011) | 11 August 20111 (11.08.2011) |
| Name and mailing address of the ISA/<br>RU<br><br>Facsimile No. | Authorized officer<br><br>Telephone No. |

Form PCT/ISA/210 (second sheet) (April 2005)

**INTERNATIONAL SEARCH REPORT**

International application No.
PCT/RU 2010/000694

C (Continuation). DOCUMENTS CONSIDERED TO BE RELEVANT

| Category* | Citation of document, with indication, where appropriate, of the relevant passages | Relevant to claim No. |
|---|---|---|
| Y | Bhuvanesh Dave et al. Tp53-associated growth arres and DNA damage repair gene expression is attenuated in mammary epithelial cells of rats fed whey proteins. J. Nutr., 2006, 136: 1156-1160, especially, the abstract, page 1157, left column, figures 1-4 | 4,9,22 |
| Y | US 5 854 224 A (COMMONWEALTH SCIENTIFIC et al) 29.12.1998, columns 2,6 | 15,33 |
| Y | US 2003/0088366 A1 (NEUROCRINE BIOSCIENCES, INC.) 08.05.2003, paragraph [0050] | 18,36 |
| Y | Robert Haner et al. The sequence-specific cleavage of RNA by artificial chemical ribonucleaes. Antisense & Nucleic acid drug development, 1997, 7:423-430 | 10,11,28,29 |
| Y | Robert E. Canfield. Peptides derived from tryptic digestion of egg white lysozyme. The Jour nal of Biological Chemistry, 1963, vol. 238, No 8: 2691-2697, the abstract | 1-36 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5495423 A
- US 6226603 B1
- US 5854992 A

### Non-patent literature cited in the description


- **LUNDSTROM K.** Structural genomics and drug discovery. *J Cell Mol Med,* March 2007, vol. 11 (2), 224-38
- **MA B ; NUSSINOV R.** Trp/Met/Phe hot spots in protein-protein interactions: potential targets in drug design. *Curr Top Med Chem.,* 2007, vol. 7 (10), 1007-13
- **KESKIN O ; GURSOY A ; MA B ; NUSSINOV R.** Towards drugs targeting multiple proteins in a systems biology approach. *Curr Top Med Chem.,* 2007, vol. 7 (10), 945-53
- **SHCHEKOTIKHIN A.E. ; DEZHENKOVA L.G. ; SUSOVA O.Y. ; GLAZUNOVA V.A. ; LUZIKOV Y.N. ; SINKEVICH Y.B. ; BUYANOV V.N. ; SHTIL A.A. ; PREOBRAZIRENSKAYA M.N.** Naphthoindole-based analogues of tryptophan and tryptamine: synthesis and cytotoxic properties. *Bioorg. Med. Chem,* 2007, vol. 15, 2651-2659
- **JEAN-MARIE LEHN.** Supramolecular Chemistry. Concepts and Perspectives. VCH Verlagsgesellschaft mbH, 1995, 103
- **BUTCHER LA ; TOMKINS JK.** A comparison of silver staining methods for detecting proteins in ultrathin polyacrylamide gels on support film after isoelectric focusing. *Anal Biochem,* 1985, vol. 1, 384-8
- **MELEN K ; RONNI T ; BRONI B ; KRUG RM ; VON BONSDORFF CH ; JULKUNEN I.** Interferon-induced Mx proteins form oligomers and contain a putative leucine zipper. *J Biol Chem.,* 1992, vol. 267, 25898-907
- **GALASSO M, ELENA ; SANA M ; VOLINIA S.** Non-coding RNAs: a key to future personalized molecular therapy?. *Genome Med.,* 18 February 2010, vol. 2 (2), 12
- **JUNG J ; SOLANKI A ; MEMOLI KA ; KAMEI K ; KIM H ; DRAHL MA ; WILLIAMS LJ ; TSENG HR ; LEE K.** Selective inhibition of human brain tumor cells through multifunctional quantum-dot-based siRNA delivery. *Angew Chem Int Ed Engl,* 2010, vol. 49 (1), 103-7
- **LI X ; LIU Y ; WEN Z ; LI C ; LU H ; TIAN M ; JIN K ; SUN L ; GAO P ; YANG E.** Potent anti-tumor effects of a dual specific oncolytic adenovirus expressing apoptin in vitro and in vivo. *Mol Cancer.,* 20 January 2010, vol. 9, 10
- **EMMRICH S ; WANG W ; JOHN K ; LI W ; PÜTZER BM.** Antisense gapmers selectively suppress individual oncogenic p73 splice isoforms and inhibit tumor growth in vivo. *Mol Cancer,* 11 August 2009, vol. 8, 61
- **FISHER M ; ABRAMOV M ; VAN AERSCHOT A ; ROZENSKI J ; DIXIT V ; JULIANO RL ; HERDEWIJN P.** Biological effects of hexitol and altritol-modified siRNAs targeting B-Raf. *Eur J Pharmacol.,* 15 March 2009, vol. 606 (1-3), 38-44
- **CZAUDERNA F ; FECHTNER M ; DAMES S ; AYGÜN H ; KLIPPEL A ; PRONK GJ ; GIESE K ; KAUFMANN J.** Structural variations and stabilising modifications of synthetic siRNAs in mammalian cells. *Nucleic Acids Res.,* 01 June 2003, vol. 31 (11), 2705-16
- **CROOKE RM ; GRAHAM MJ ; MARTIN MJ ; LEMONIDIS KM ; WYRZYKIEWIECZ T ; CUMMINS LL.** Metabolism of antisense oligonucleotides in rat liver homogenates. *J Pharmacol Exp Ther.,* January 2000, vol. 292 (1), 140-9
- **MONIA BP ; JOHNSTON JF ; SASMOR H ; CUMMINS LL.** Nuclease resistance and antisense activity of modified oligonucleotides targeted to Ha-ras. *J Biol Chem.,* 14 June 1996, vol. 271 (24), 14533-40
- **GILES RV ; SPILLER DG ; GREEN JA ; CLARK RE ; TIDD DM.** Optimization of antisense oligodeoxynucleotide structure for targeting ber-abl mRNA. *Blood,* 15 July 1995, vol. 86 (2), 744-54
- **XODO L ; ALUNNI-FABBRONI M ; MANZINI G ; QUADRIFOGLIO F.** Pyrimidine phosphorothioate oligonucleotides form triple-stranded helices and promote transcription inhibition. *Nucleic Acids Res.,* 25 August 1994, vol. 22 (16), 3322-30
- **SAZANI P ; WELLER DL ; SHREWSBURY SB.** Safety pharmacology and genotoxicity evaluation of AVI-4658. *Int J Toxicol.,* March 2010, vol. 29 (2), 143-56

- **MOURICH DV ; JENDRZEJEWSKI JL ; MARSHALL NB ; HINRICHS DJ ; IVERSEN PL ; BRAND RM.** Antisense targeting of cFLIP sensitizes activated T cells to undergo apoptosis and desensitizes responses to contact dermatitis. *J Invest Dermatol.,* August 2009, vol. 129 (8), 1945-53
- **BELON CA ; FRICK DN.** Helicase inhibitors as specifically targeted antiviral therapy for hepatitis C. *Future Virol.,* 01 May 2009, vol. 4 (3), 277-293
- **BAGNYUKOVAL T.V. ; POGRIBNY I.P. ; CHEKHUN V.F.** MicroRNAs in normal and cancer cells: A New class of gene expression regulators. *Experimental Oncology,* 2006, vol. 28, 263-269
- **STEINHAUSER I ; LANGER K ; STREBHARDT K ; SPÄNKUCH B.** Uptake of plasmid-loaded nanoparticles in breast cancer cells and effect on Plk1 expression. *J Drug Target,* September 2009, vol. 17 (8), 627-37
- **LU JJ ; LANGER R ; CHEN J.** A novel mechanism is involved in cationic lipid-mediated functional siRNA delivery. *Mol Pharm,* May 2009, vol. 6 (3), 763-71
- **MOREIRA JN ; SANTOS A ; MOURA V ; PEDROSO DE LIMA MC ; SIMÕES S.** Non-viral lipid-based nanoparticles for targeted cancer systemic gene silencing. *J Nanosci Nanotechnol,* May 2008, vol. 8 (5), 2187-204
- **LANFORD R.E. ; BUTEL J.S.** *Cell.,* 1984, vol. 37, 801-813
- **ROBBINS, J. B. ; R. SCHNEERSON ; S. C. SZU.** Perspective: hypothesis: serum IgG antibody is sufficient to confer protection against infectious disease by inactivating the inoculum. *J. Infect. Dis,* 1995, vol. 171, 1378-1398
- **DEL VAL, M. ; H. J. SCHLICHT ; H. VOLKMER ; M. MESSERLE ; M. J. REDDEHASE ; U. H. KOSZINOWSKI.** Protection against lethal cytomegalovirus infection by a recombinant vaccine containing a single nonameric T-cell epitope. *J. Virol,* 1991, vol. 65, 3641-3646
- **LARSEN, D. L. ; A. KARASIN ; C. W. OLSEN.** Immunization of pigs against influenza virus infection by DNA vaccine priming followed by killed-virus vaccine boosting. *Vaccine,* 2001, vol. 19, 2842-2853
- **CICIN-SAIN, L. ; BRUNE, W. ; BUBIC, I. ; JONJIC, S. ; KOSZINOWSKI, U. H.** Vaccination of Mice with Bacteria Carrying a Cloned Herpes Virus Genome Reconstituted In Vivo. *J. Virol.,* 2003, vol. 77, 8249-8255
- **LEVIN SA ; DUSHOFF J ; PLOTKIN JB.** Evolution and persistence of influenza A and other diseases. *Math Biosci.,* March 2004, vol. 188, 17-28
- **TERREGINO C ; TOFFAN A ; BEATO MS ; DE NARDI R ; DRAGO A ; CAPUA I.** Conventional H5N9 vaccine suppresses shedding in specific-pathogen-free birds challenged with HPAI H5N1 A/chicken/Yamaguchi/7/2004. *Avian Dis.,* March 2007, vol. 51 (1), 495-7
- **MEDVEDEVA MN ; PETROV NA ; VASILENKO SK ; SIMANOVSKAIA VK ; GOLUBEV DB.** The characteristics of the hemagglutinin from persistent variants of the influenza virus A/Victoria/35/72 (H3N2. *Vopr Virusol.,* September 1990, vol. 35 (5), 374-6
- **ARONSSON F ; ROBERTSON B ; LJUNGGREN HG ; KRISTENSSON K.** Invasion and persistence of the neuroadapted influenza virus A/WSN/33 in the mouse olfactory system. *Viral Immunol.,* 2003, vol. 16 (3), 415-23
- **COX MM.** Vaccines in development against avian influenza. *Minerva Med.,* April 2007, vol. 98 (2), 145-53
- **GRONVALL GK ; BORIO LL.** Removing barriers to global pandemic influenza vaccination. *Biosecur Bioterror.,* 2006, vol. 4 (2), 168-75
- **TAUBENBERGER JK ; MORENS DM ; FAUCI AS.** The next influenza pandemic: can it be predicted?. *JAMA,* 09 May 2007, vol. 297 (18), 2025-7
- **VARDAVAS R ; BREBAN R ; BLOWER S.** Can influenza epidemics be prevented by voluntary vaccination?. *PLoS Comput Biol,* 04 May 2007, vol. 3 (5), e85